(19) Europäisches Patentamt / European Patent Office / Office européen des brevets

(11) **EP 4 264 248 B1**

(12) **EUROPEAN PATENT SPECIFICATION**

(45) Date of publication and mention
of the grant of the patent:
**28.01.2026 Bulletin 2026/05**

(21) Application number: **21844138.4**

(22) Date of filing: **17.12.2021**

(51) International Patent Classification (IPC):
***G01N 27/414*** (2006.01)   ***B01L 3/00*** (2006.01)
***G01N 33/487*** (2006.01)   ***G01N 35/00*** (2006.01)

(52) Cooperative Patent Classification (CPC):
**G01N 27/414; B01L 3/502715; B01L 7/00;**
**G01N 15/1023; G01N 15/1031; G01N 15/1433;**
**G01N 15/1484; G01N 33/48728; G02B 21/28;**
**G02B 21/34;** B01L 2200/0668; B01L 2200/147;
B01L 2300/027; B01L 2300/0663; B01L 2300/0829;
(Cont.)

(86) International application number:
**PCT/US2021/064009**

(87) International publication number:
**WO 2022/140174 (30.06.2022 Gazette 2022/26)**

(54) **SYSTEMS, DEVICES AND METHODS FOR CELL ANALYSIS USING CHEMFET SENSOR ARRAYS**

SYSTEME, VORRICHTUNGEN UND VERFAHREN ZUR ZELLANALYSE MIT CHEMFET-SENSORANORDNUNGEN

SYSTEMES, DISPOSITIFS ET METHODES D'ANALYSE DE CELLULES UTILISANT DES RESEAUX DE CAPTEURS CHEMFET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2020 US 202063128751 P**

(43) Date of publication of application:
**25.10.2023 Bulletin 2023/43**

(73) Proprietor: **Life Technologies Corporation**
**Carlsbad, CA 92008 (US)**

(72) Inventors:
• **BEACHAM, Daniel W.**
**Carlsbad, California 92008 (US)**
• **HINZ, Wolfgang**
**Carlsbad, California 92008 (US)**
• **PARKER, Scott T.**
**Carlsbad, California 92008 (US)**
• **DONOHUE, John**
**Carlsbad, California 92008 (US)**

(74) Representative: **HGF**
**HGF Limited**
**4th Floor, 1 City Square**
**Leeds LS1 2AL (GB)**

(56) References cited:
JP-A- 2013 096 868      US-A1- 2010 301 398
US-A1- 2012 143 531     US-A1- 2019 264 274
US-A1- 2020 088 676

• **SUDIBYA HERRY?GUNADI ET AL: "Interfacing Glycosylated Carbon-Nanotube-Network Devices with Living Cells to Detect Dynamic Secretion of Biomolecules", ANGEWANDTE CHEMIE INTERNATIONAL EDITION, vol. 48, no. 15, 4 March 2009 (2009-03-04), pages 2723 - 2726, XP055907645, ISSN: 1433-7851, DOI: 10.1002/ anie.200805514**

• **HU YUANQI ET AL: "3-T ISFET front-end utilising parasitic device capacitance", ELECTRONICS LETTERS, THE INSTITUTION OF ENGINEERING AND TECHNOLOGY, GB, vol. 50, no. 21, 9 October 2014 (2014-10-09), pages 1507 - 1509, XP006049679, ISSN: 0013-5194, DOI: 10.1049/ EL.2014.2488**

(52) Cooperative Patent Classification (CPC): (Cont.)
B01L 2300/1827; B01L 2300/185; B01L 2300/1894;
G01N 2015/1006

## Description

### CROSS REFERENCE TO RELATED APPLICATIONS

This application claims benefit of U.S. Provisional Application No. 63/128,751 filed December 21, 2020.

### OVERVIEW

Electrophysiological and metabolic phenotyping of cell behaviors remain crucial avenues of research in modern cell biology with large unmet needs for researchers in areas of, for example, engineered tissues, primary cell types and oncology discovery. Successful measurements of cellular excitability are currently hindered by cumbersome patch clamp and microelectrode voltammetry methods, while interrogations of metabolic function remain hampered by the technical limitations of large scale population and biochemical workflows, inefficiently deployed to access crucial metrics of single cell behavior.

It is becoming more evident that the study of model systems at the single cell level would provide greater insight into normal cell physiology and alterations during pathological conditions such as cancer, diabetes and neurodegenerative disease. Recent applications that highlight the advantages of single cell studies vs. cell population studies include, for example, studies of transcriptomics and aging, studies of signaling pathways within cells and bioenergetic measurements of metabolism.

Accordingly, systems, devices and methods of the present disclosure can provide an end user with real-time cell analysis and imaging of single cells in a population of cells. Cell analysis systems of the present disclosure can measure the electrical and metabolic activity of single, living cells with subcellular addressability and simultaneous data acquisition for between, for example, about 10 cells to about 100,000 cells in a single analysis. Cell analysis systems of the present disclosure can provide electroscopic imaging, which is an image of cellular response as detected by ChemFET sensors, for example an image of cellular response from sensors covering a cell footprint or an image of cellular response for sensors detecting cellular effluent.

Additionally, cell analysis systems of the present disclosure can provide optical imaging, which can be compared to electroscopic imaging. In order to provide a cell-compatible fluid environment, cell analysis systems of the present disclosure can include an automated fluidic system that provides an end-user control over selection and flow parameters for reagent delivery to cells in an analysis chamber, as well as selection of gases in equilibrium with various reagents and solutions. Additionally, user-selected precision temperature control of ChemFET devices, reagents and solutions, as well as the analysis compartment provide consistency in the cell analysis system environment.

US 2020/0088676 A1 describes a ChemFET-array-based cell analysis system for measuring the electrical and metabolic activity of a single, living cell with sub-cellular addressability. The sensors of the sensor array have a pitch of between 3.36 $\mu$m to about 850 nm.

### BRIEF DESCRIPTION OF THE DRAWINGS

The novel features of the present disclosure are set forth in the appended claims.

Claim 1 defines a sensor system and claim 12 refers to a flow cell device comprising the sensor device.

A better understanding of the features and advantages of what is disclosed herein will be obtained by reference to the following detailed description that sets forth illustration of the features and advantages of systems, devices and methods for cell analysis, in which the principles of the present disclosure are utilized, and the accompanying drawings of which:

FIG. 1A is a block diagram that illustrates generally various components of a cell analysis system of the present disclosure.

FIG. 1B is a block diagram that illustrates generally the integration of various elements of a fluidic system with a chip interface device and a chip device of the present disclosure.

FIG.2 is an exploded view that illustrates generally an a sensor array device used in a cell analysis system of the present disclosure.

FIG. 3 is a perspective section view of a sensor array device that illustrates generally various aspects of a portion of a flow cell chamber defined by a sensor array device.

FIG. 4A is a perspective view that illustrates generally an a sensor array device used in a cell analysis system of the present disclosure capable of providing various segments of a ChemFET for plating cell samples. FIG. 4B is an exploded view of an upper section of FIG. 4A.

FIG. 5A illustrates generally an electrical model of a cell on a ChemFET sensor array of the present disclosure.

FIG. 5B is a schematic section view of a cell on a sensor chip that illustrates generally structural components of a ChemFET sensor without microwells used for cell analysis.

FIG. 5C is a schematic section view of a cell on a sensor chip that illustrates generally structural components of a ChemFET sensor with microwells used for cell analysis.

FIG. 6A is a schematic representation that illustrates generally a chip clamp assembly of a cell analysis system of the present disclosure.

FIG. 6B is a schematic representation that illustrates generally a flow cell formed between a chip interface device and a chip device of the present disclosure.

FIG. 7 is an exploded perspective view that illustrates generally a chip clamp assembly of a cell analysis system of the present disclosure.

FIG. 8A is a schematic representation that illustrates generally a chip clamp assembly of a cell analysis system of the present disclosure with integrated microscope assembly.

FIG. 8B is a schematic representation that illustrates generally a flow cell formed between a chip interface device and a chip device of the present disclosure.

FIG. 9A is an exploded perspective view that illustrates generally a chip clamp assembly of the present disclosure for use with an integrated microscope assembly.

FIG. 9B is a perspective section view that illustrates generally alignment of a microscope objective over a sensor array device of the present disclosure.

FIG. 10A is a side perspective view that illustrates generally a chip clamp assembly of the present disclosure for use with an integrated microscope assembly.

FIG. 10B is a bottom perspective view that illustrates generally a chip clamp assembly of the present disclosure for use with an integrated microscope assembly.

FIG. 10C is an exploded perspective view that illustrates generally a chip clamp assembly of the present disclosure for use with an integrated microscope assembly.

FIG. 11 is an exploded view that illustrates generally a section of a chip interface device of the present disclosure in relationship to a chip device.

FIG. 12A and FIG. 12B are exploded views that illustrate generally a section of a chip interface device with optical interface in relationship to various subassemblies of the present disclosure.

FIG. 13 is a perspective view that illustrates generally a cell analysis system of the present disclosure.

FIG. 14A is a graph of the temperature maintained for a thermoelectric cooling (TEC) device over a time interval, while FIG. 14B is a graph of the temperature of effluent solution as measured in a waste line over the time interval of FIG. 15A.

FIG. 15 is a set of graphs comparing target setpoint temperature of a TEC versus measured temperature for a TEC in a chip thermal control assembly of the present disclosure.

FIG. 16A is a micrograph of HEPG2 cells on an exemplary sensor array device, while FIG. 16B is a micrograph of HEPG2 cells on conventional microscope slide.

FIG. 17A is the micrograph of HEPG2 cells on an exemplary sensor array device, while FIG. 17B is an electroscopic image of HEPG2 cells on a sensor array device.

FIG. 18A and 18B are graphs of time course studies of glycolysis using HEPG2 cells.

FIG. 19A and 19B are graphs of time course studies of glycolysis using A637 cells.

FIG. 20 is a graph of responses of 3 individual cells from a selected time interval of the study presented in FIG. 19A versus the average population response.

FIG. 21 is an electroscopic image of the cells of FIG. 20.

FIG. 22A and FIG. 22B are electroscopic images of cardiomyocytes at two sequential time points.

FIG. 22C is the graph of responses for 3 cells selected from the electroscopic images of FIG. 22A and FIG. 22B.

## DETAILED DESCRIPTION

Systems, devices and methods of the present disclosure can provide an end user with real-time cell analysis and imaging of single cells in a population of cells. Cell analysis systems of the present disclosure can measure the electrical and metabolic activity of single, living cells with subcellular addressability and simultaneous data acquisition for between, for example, about 10 cells to about 100,000 cells in a single analysis.

Cell analysis systems of the present disclosure can provide electroscopic imaging, which is an image of cellular response as detected by ChemFET sensors, for example an image of cellular response from sensors covering a cell footprint or an image of cellular response for sensors detecting cellular effluent. Additionally, cell analysis systems of the present disclosure can provide optical imaging, which can be compared to electroscopic imaging. Cell analysis systems of the present disclosure can include an automated fluidic system that provides an end-user control over selection of reagents and flow parameters for reagents delivered to cells in an analysis chamber, as well as selection of gases in equilibrium with various reagents and solutions. Additionally, user-selected precision temperature control of ChemFET devices, reagents and solutions, as well as the analysis compartment provide consistency in the cell analysis system environment.

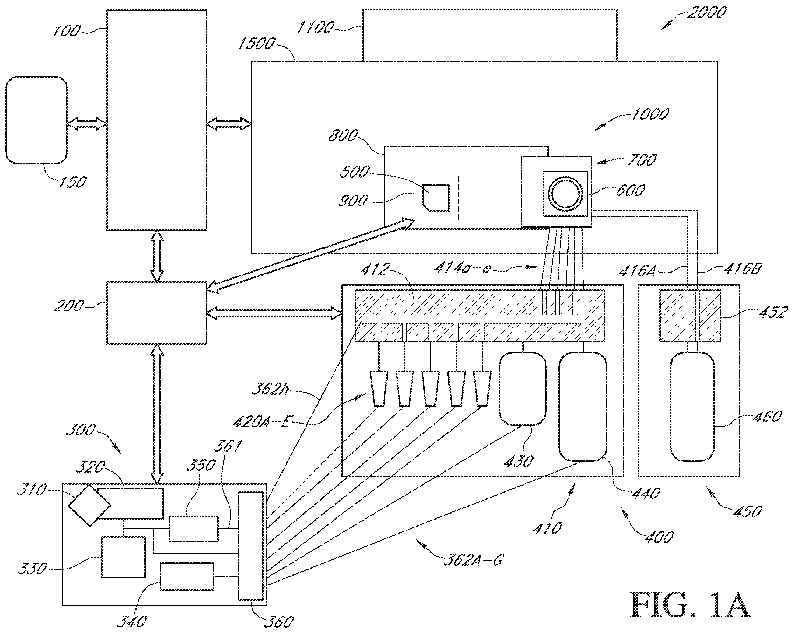

FIG. 1A is a block diagram that illustrates generally various components of a cell analysis system of the present disclosure. Cell analysis system 2000 can include chip clamp assembly 1000 in cell analysis compartment 1500. Various cell analyses performed using cell analysis system 2000 are performed using a sensor array chip, which is part of sensor array device 500. As recited herein, the terms "sensor" and "pixel" can be used interchangeably, as can as well as "sensor array" and "ChemFET sensor array". Additionally, the terms "sensor array chip," "sensor array device ","sensor device", "sensor chip," "chip," and derivatives of these terms can be used interchangeably. For example, sensor array device 500 is also referred to as chip device 500, and sensor device 500.

Chip interface device 600, mounted to chip interface device shuttle 700, has a chip device interface component, which provides a top flow cell cover for chip device 500 that reversibly seals a top surface of chip device 500 to functionally provide a flow cell; operatively also an analysis chamber. As will be described in more detail herein, when a top flow cell cover is optically transparent, various chip interface devices also have an optical interface component that provides for an interface with microscope assembly 1100.

Additionally, chip interface device 600 has a fluidic interface component including a fluidic interface block that is an interface between fluid system 400 and a microfluidic circuit of chip interface device 600, which can be in fluid communication with chip device 500. Fluidic system 400 can be configured by an end user to execute a sequence of fluidic operations for the sequential delivery of various solutions to sensor array device 500 over the course of a cell analysis experiment. In that regard, chip interface device 600 provides a the macro-to-micro fluidic interface between fluidic system 400 and chip device 500.

Exemplary fluidic operations include washing, priming and reagent delivery through the fluidic circuit of chip interface device 600. The microfluidic circuit, also referred to as a microfluidic multiplexer circuit, is configured to provide fluid distribution through the flow cell defined by the sealed top cover provided by chip interface device 600. During an analysis, activity of cells plated on a sensor device surface can be detected by the sensor array device defining the bottom surface of the flow cell/analysis chamber. Using a microfluidic multiplexer circuit for fluid distribution to a sensor device for a sequence of fluidic operations during a cell analysis experiment can avoid cross-contamination of solutions used during analysis in various fluidic compartments, as well as providing sharp transitions between reagent fluid streams used during an analysis.

Chip interface device shuttle 700 of chip clamp assembly 1000 is mounted to chip clamp base 800. Once chip device 500 has been loaded in chip clamp assembly 1000, chip interface device shuttle 700 can be moved on a linear positioning system so that chip interface device 600 is positioned over chip device 500. Chip thermal control assembly 900 is mounted beneath chip device 500, as indicated by the hatched line. As will be described in more detail herein, chip thermal control assembly 900 includes a thermoelectric cooling (TEC) device in contact with an aluminum base on one side, and in contact with a cooling assembly on the opposite side of the TEC. With the application of a clamping force to chip interface device 600, chip interface device 600 can be engaged to provide contact between chip device 500 and electronic connections, as well as ensuring contact of the TEC with the temperature control assembly. With respect to providing chip thermal control that can enable a wide variety of cell analysis protocols, chip thermal control assembly 900 can provide precision thermostatting of chip device 500 over a temperature range of 4°C to 60°C within +/- 0.1 C. In addition to providing engagement with electrical and thermal control components with respective devices and assemblies, application of a clamping force also provides that chip interface device 600 can be positioned to be sealably engaged with and reversibly coupled to sensor array device 500, thereby forming a flow cell, which is operatively also an analysis chamber.

Fluidic system 400 of FIG. 1A can include reagent and solution compartment 410 and waste compartment 450. As depicted in FIG. 1A, chip interface device 600 can be in fluid communication with fluidic manifold 412 in reagent and solution compartment 410 of fluidic system 400. Fluidic manifold 412 can be in fluid communication with a bank of reagent and solution containers, 420A-420E. Additionally, solutions used for cleaning the fluidic system can be provided in cleaning solution container 430, while solutions used in high volume, such as various buffered solutions used in cell culture and cell analysis, for example, phosphate buffered saline (PBS), Ringer's solution, and the like, can be provided in wash solution container 440. As will be described in more detail herein, reagent and solution compartment 410 can be maintained at an end user selected temperature to maintain reagents and solutions housed in reagent and solution compartment 410 at a desired temperature. To provide a range of temperatures at which the reagents and solutions can be maintained for a variety of cell analyses, reagent and solution compartment 410 can be maintained at an end user selected temperature of between about 10°C to about 50°C. Accordingly, reagent and solution compartment 410 can be maintained at an end user selected temperature of between about 20°C to about 37°C, which is a range in which many cell-based assays are conducted. Finally, chip and bulk waste lines 416A-416-B can be in fluid communication with waste container 460 of waste compartment 450 with control provided by waste manifold 452.

With respect to motive force for moving fluids through the cell analysis system, pneumatic system 300 can be in fluid communication with fluidic system 400. Pneumatic manifold 400 can include filter 310, and compressor 320 for creating compressed clean dry air (CDA) when the source gas is air. Compressed CDA can be stored in compressed CDA reservoir 330, which can be, for example, a cylinder. Compressed CDA reservoir 330 can be in fluid communication with regulator 350, which can be in fluid communication with pneumatic manifold 360. Pressure sensors 340 monitor the pressure in pneumatic manifold 360, which feeds back into a control system for controlling pneumatic manifold during a run. Additionally, compressed gas sources of a user specified composition for various cell analyses can be placed in fluid communication directly with regulator 350 and therefore in fluid communication with pneumatic manifold 360. For example, cells growing in bicarbonate buffered cell culture medium require a compressed air composition that includes 5% carbon dioxide. Alternatively, for studies of cell hypoxia, ambient oxygen levels in the gas source can be varied from normal levels of 20% oxygen down to 1% .

As depicted in FIG. 1A, pneumatic manifold lines 362A-363E from pneumatic manifold 300 are in fluid communication with each of reagent containers, 420A-420E, respectively. Similarly, pneumatic manifold lines 362F and 363G are in fluid communication with cleaning solution container 430, and wash solution container 440, respectively. Pneumatic manifold line 362H, shown in FIG. 1A to be in fluid communication with fluidic manifold 412, can be used to purge the lines of cell analysis system 2000 with CDA, for example, during a system cleaning procedure. Valve board 200, controlled by processing electronics assembly 100, controls the valving for both pneumatic manifold 360 and fluidic manifold 412, as well as for chip thermal control assembly 900. Accordingly, an end user can specify the selection of a reagent or solution, the gas composition in equilibrium with a reagent or solution, as well as the sequence, the flow rate, flow duration, and the measurement protocol during the flow of a selected reagent or solution.

Pneumatic system 300, controlled by processing electronics assembly 100, provides a defined and controllable pressure difference between source containers, such as reagent containers, 420A-420E, cleaning solution container 430, and wash solution container 440, and an output container, such as waste container 460 of waste compartment 450. As depicted in FIG. 1A, source containers 420A-420-E are in fluid communication with chip interface device 600 via fluidic manifold input lines 414A-414E. Similarly, waste effluent, such as chip waste effluent and bulk waste effluent, are in fluid communication with waste container 460 via fluidic manifold output lines 416A and 416B. As such, a defined and controlled pressure difference provides a defined and controlled flow rate of various selected reagents and solutions through analysis system 2000 over the course of an analysis. For cell analysis system 2000, flow rates of 10 $\mu$l/sec to 500 $\mu$l/sec can be selected.

Processing electronics assembly 100 can include components such as a reader board and a processor complex, as well as responsive to interactive end user input through user display 150. Processing electronics assembly 100 can include an array controller, which can provide various power supplies and bias voltages, control and timing signals to sensor array device 500, as well as provide a data and processor interface for high-speed acquisition of data from sensor array device 500. In addition to including an array controller, processing electronics assembly 100 can control the valve board and fluidic processing of cell analysis system 2000, as well as manage setup of experiments, storage of sensor output from experiments, generate log files for experiment results and instrument operation, and process experiment output data for presentation to the user.

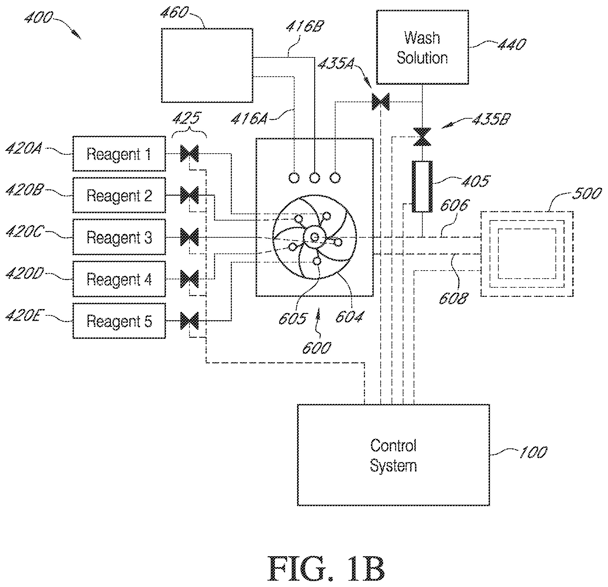

FIG. 1B is a block diagram that illustrates generally the integration of various elements of a fluidic system 400 with chip interface device 600 and chip device 500. As depicted in FIG. 1A, reagent and solution containers 420A-420E of fluidic system 400, are in fluid communication with chip interface device 600. During use, sensor array device 500 is sealably attached to chip interface device 600. Microfluidic multiplexer circuit 604 of chip interface device 600 permits various user-selected reagents and solutions from reagents and solution containers 420A-420E to be controllably delivered to sensor array device 500 via reagent values 425, which are controlled through processing electronic assembly 100 via valve board 200 of FIG. 1A. A selected reagent or solution can be placed in fluid communication with each of an inlet port, such as fluidic multiplexer circuit inlet ports 605 of FIG. 1B, then directed to sensor array device 500 via chip interface device inlet channel 606 of chip interface device 600. Effluent flowing from sensor array device 500 is returned to chip interface device 600 via chip interface device outlet channel 608, which can be placed into fluid communication with any of fluidic manifold fluidic manifold waste lines 416A-416B to waste container 460. Accordingly, reagents and solutions from the fluidic system 400 can be selectively driven through chip interface device 600 by control of reagent valves 425, which receive signals from processing electronics assembly 100, and then through sensor array device 500 to waste container 460.

Processing electronics assembly 100 of FIG. 1B also includes controllers for wash solution valves 425A and 425B, as well as for reference electrode 405. Reference electrode 405 is an important component for providing a stable reference voltage to a cell analysis system, as each sensor of a sensor array device generates an output signal that depends on the value of a stable reference voltage. As depicted in FIG. 1B, reference electrode 405 is in a flow path of wash solution from wash solution container 440, and in contact with sensor device 500 via chip interface device 600. The constant electrolyte fluidic environment of reference electrode 405 provides a constant and stable reference voltage to sensor array device 500. As depicted in FIG. 1B, a tee in the wash solution line provides a controllable flow path for wash solution to microfluidic multiplexer circuit 604, as well as to chip interface device inlet channel 606. When a reagents or solution from reagents and solution containers 42A-420E is controllably selected, wash solution valve 435B is closed with reference electrode 405 in contact with the wash solution. If wash solution is the solution selected, the valve wash solution valve 435A is opened. During the delivery of a reagent or solution through the system, the reference electrode 405 remains in constant contact with wash solution, and a stable reference potential is applied to sensor array device 500. According to the present disclosure, reference electrode 405 can be a hollow cylindrical structure, for example, of an inert metal, non-limiting examples of which include platinum or titanium. Such hollow cylindrical metal structures, can provide effective ohmic contact with a fluid in a flow stream.

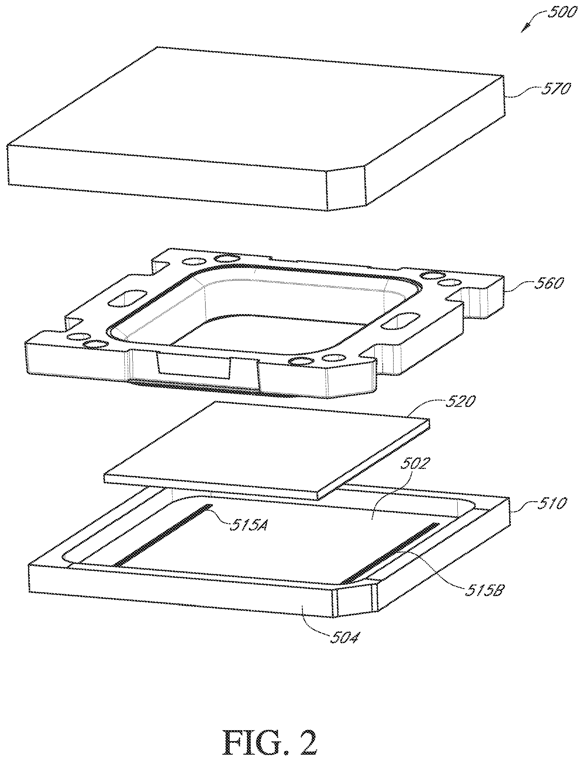

FIG. 2 is an exploded view that illustrates generally sensor array device 500 used in a cell analysis system 2000. As depicted in FIG. 2, sensor array device 500 includes chip device substrate 510, upon which sensor chip 520 is mounted on first device substrate surface 502. Chip device substrate 510 includes a first set 515A and a second set 515B of electronic connections formed through chip device substrate 510, with pogo pin contact pads on second device substrate surface 504. Sensor chip 520 can be engage with a pogo pin block, and in turn, the pogo pin block can be engaged with a printed circuit (PC) board mounted to chip clamp base 800 of FIG. 1A. The integrated circuit connections provide power, timing and control to, as well as data acquisition from a sensor chip by a cell analysis system. The PC board is controlled by processing electronics assembly 100 of FIG. 1A.

Fluid dam 560 is a frame that is sealably mounted to sensor chip 520. As chip device 500 has an exposed sensor array surface, chip device cover 570 is a removable cover fitting over fluid dam 560 that can cover chip device 500 during handling to protect the sensor array surface until it is used. Given that the structure of fluid dam 560 is an essential element in defining a flow cell, fluid dam 560 is sealed against sensor chip 520 to prevent leakage at the interface between fluid dam 560 and sensor chip 520 during analysis, thereby preventing the shorting of electrical connections between sensor chip 520 and chip device substrate 510, as well as other operational electrical connections of sensor array device 500 to a cell analysis system. Fluid dam 560 is a frame that can be made from a variety of polymeric materials that are chemically inert material and resistant to swelling in aqueous-based solutions over a pH range of 5-9, as well as aqueous solutions that can include organic solvents, surfactants, and chelating agents. Some exemplary materials include, for example, but not limited by, nylon 6/6, nylon 6, nylon 10, polypropylene, polyarylamide, polycarbonate, polystyrene, polyethylene, polysulfone, and polyphenylene sulfide.

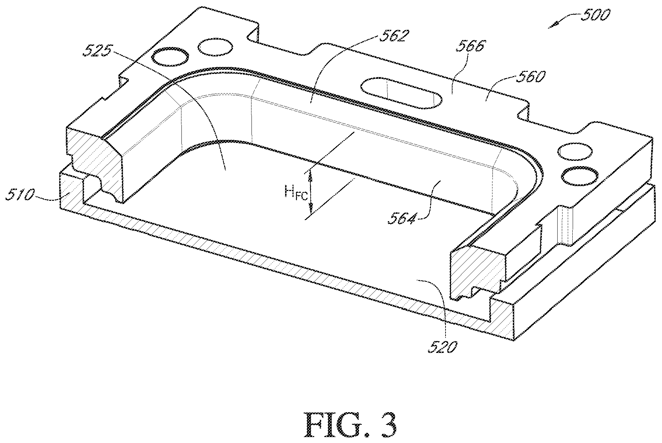

FIG. 3 is a perspective section view of sensor array device 500 that illustrates generally sensor chip 520 mounted upon chip device substrate 510 with fluid dam 560 mounted upon sensor chip 520. Fluid dam 560 has features that, in conjunction with features of a mating surface of a chip interface device, set the height of the flow cell chamber (analysis chamber) formed between the upper surface of sensor chip 520 and a top surface defining a flow cell cover. Fluid dam 560 has chamfer 562, with chamfer upper ridge 561 and chamfer lower ridge 563, as well as fluid dam top or upper surface 566. Additionally, fluid dam 560 has fluid dam inner wall surface 564, defined by the height, $H_{AC}$, which is dimensioned to provide a sealing surface for the seating and sealing of a flow cell top cover. As will be described in more detail herein, a mating surface of structure of a chip interface device can engage with and reversibly couple to fluid dam 560, so that an O-ring seal is positioned below chamfer lower ridge 563, with the O-ring seated against fluid dam inner wall surface 564 to provide a reversable seal between a flow cell top cover and a chip device. Once reversibly sealed with a flow cell top cover, a functional flow cell is defined having a flow cell chamber, or analysis chamber, with a defined height and a flow cell volume. As will be described in more detail herein, the flow cell volume can be adjusted by use of a spacer of a defined height, which can be placed between upper surface 566 of fluid dam 560 and a mating surface of a flow cell cover.

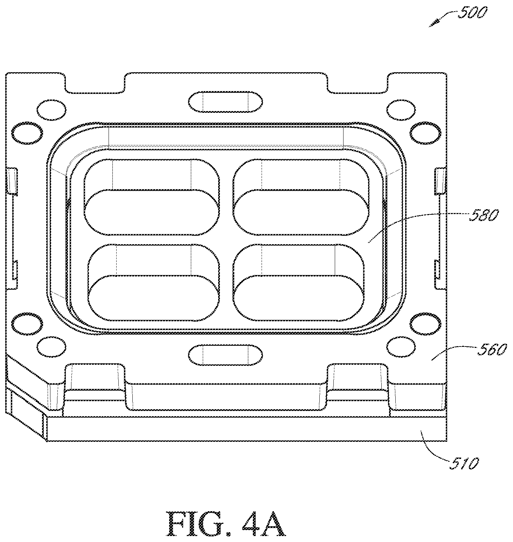

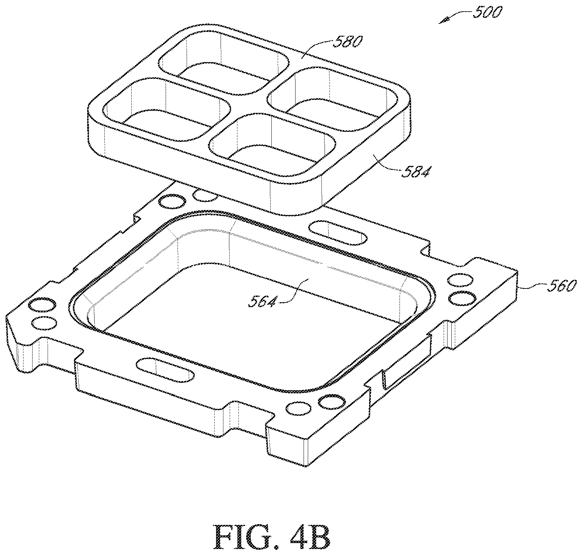

FIG. 4A is a perspective view that illustrates generally sensor array device 500 with sample divider 580, capable of providing various divided segments of a sensor array device for plating spatially divided samples of cells. FIG. 4B is an exploded perspective view of an upper section of FIG. 4A, which illustrates how sample divider perimeter wall 584 of sample divider 580 is shaped to fit inner wall surface 564 of fluid dam 560. In that regard, sample divider 580 is a conformal and removal piece that provides isolated areas on a sensor array device surface for the purpose of plating different cell samples on the same device. Though shown with four segments, sample divider can have from 2-16 segments, and can be selected from a variety of shapes. Sample divider 580 can be formed from any variety of natural or synthetic polymeric materials that are chemically inert material and resistant to swelling in aqueous environment, and capable providing a conformal sample divider. In that regard, candidate polymer materials can have a durometer rating of 0-60 on the Shore A scale, or 20-90 on the Shore OO scale. Some exemplary materials include, for example, but not limited by, silicone polymers, such as polydimethylsiloxane (PDMS), thermoplastic polyurethanes, thermoplastic Styrene-Butadiene-Styrene (SBS) polymers, such as Sofprene T®, and thermoplastic vlucanizates, such as Santoprene™.

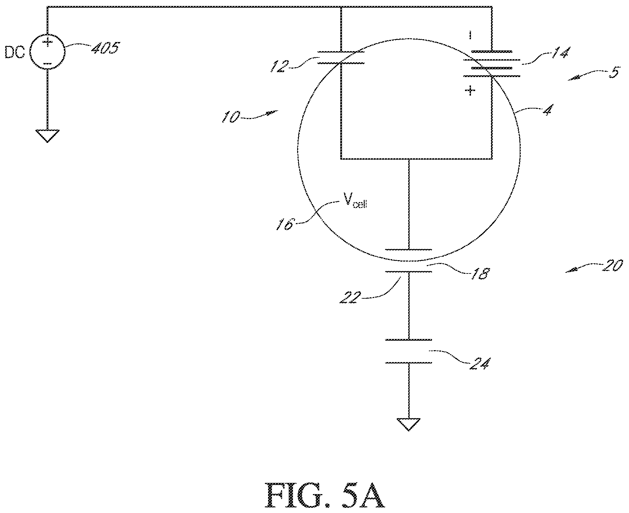

As depicted in FIG. 5A, cell electrical model 10 includes cell 5 has internal potential $V_{cell}$, while cell membrane 4 can be modeled as capacitor 12, $C_M$, in parallel to battery 14. Cell 5 is capacitively coupled to ChemFET sensor transistor 20, having cell membrane to sensor capacitance 18, where the cell internal potential, $V_{cell}$, is detected by sensor 20 across cell membrane 4 at sensing surface 22 and output as detector response 405. Hence, changes in cell membrane potential can be detected at detector 405, where detector response can range from $20\mu V$ to $100mV$ for ChemFET sensor 20, which has input capacitance 24, $C_S$. As such, based on this model the capacitor divider formed by cell membrane to sensor capacitance 18, $C_M$, and sensor input capacitance 24, $C_S$, can be expressed as:

$$C_M/(C_M + C_S) \; \text{Eq. 1}$$

The capacitor divider of Eq. 1 is a scalar that provides insight into the impact of sensor input capacitance on detecting changes in cell membrane potential. Clearly, Eq. 1 approaches unity as the sensor input capacitance tends towards zero. Conversely, the ratio of the capacitor divider becomes smaller as sensor input capacitance increases. In that regard, the magnitude of the sensor input capacitance determines the magnitude of cell membrane potential, $V_{cell}$, that can be measured by a ChemFET sensor, $V_{cell-m}$, given by:

$$V_{cell-m} = V_{cell}[C_M/(C_M + C_S)] \text{ Eq. 2}$$

Cell membrane capacitance is typically in the range of 0.5-1.0 $\mu F/cm^2$, so that the cell membrane capacitance in contact, for example, with a 3$\mu$m diameter sensor plate would be about 35-70 fF. As will be described in more detail herein, the sensor transistors forming part of the sensor device according to the present invention are compact in size and configuration, and additionally have a very low input capacitance of between 1fF to 10 fF. By entering these values into Eq. 1, the capacitor divider ratio would be in the range of 0.78 to 0.88, so that $V_{cell-m}$ is not significantly attenuated. As such, when using various ChemFET sensor devices of the present disclosure, a range of cell membrane potentials can be measured, for example, from a 1-2mV response for induced depolarization of non-excitable cells to neuron action potentials providing detector response with amplitudes of 20 mV or higher.

Though the electrical model for the purpose of explanation presents a single sensor, as will be described in more detail herein, for various cell analysis systems of the present disclosure, cells to be analyzed are plated on the sensor array surface, with the cell membranes in contact with multiple metallic sensor plates. As sensor arrays of the present disclosure are compact in size and configuration, for example, having a pitch of 0.85$\mu$, this results in a large number, for example hundreds or even thousands, of low-input capacitance sensors in contact with a given cell. This high sensor density per area of cell contact, or high sensor density per cell footprint, allows electrical measurement and amplification of potentials that appear on a large number of separate sensor plates in direct contact with a cell membrane.

In contrast, for CMOS-and FET-based multielectrode arrays (MEAs), rather than having a direct FET connection for each sensor, multiplexers are used to connect selected sensor plates to a limited number of sensors; each sensor of larger size than sensors of the present disclosure, with such sensor arrays having substantially greater pitch than sensor arrays of the present disclosure. Additionally, such CMOS-and FET-based MEAs use high-performance, low noise amplifiers, which in combination with multiplexers, provide sensors with much higher input capacitances relative to the associated cell membrane capacitance than sensors of the present disclosure.

In that regard, by inspection of Eq. 1 and Eq. 2, the much higher input capacitance of CMOS-and FET-based MEAs results in greater attenuation of the measured cell membrane potential. The net result of using sensors with a significantly higher input capacity and substantially less sensor coverage over the footprint of a cell results in substantial attenuation of signal when sensing cell membrane potentials using CMOS-and FET-based sensor arrays. In that regard, due to the high input capacitance of CMOS-and FET-based sensors, typical neuron action potentials measured by MEAs produce a detector response in the range of 150-500 $\mu$V in contrast to neuron action potentials with amplitudes of 20 mV or higher measured by ChemFET sensors of the present disclosure.

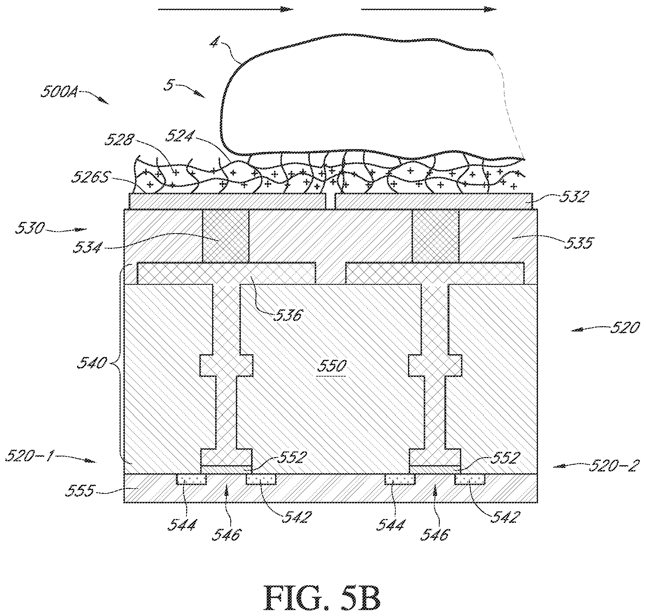

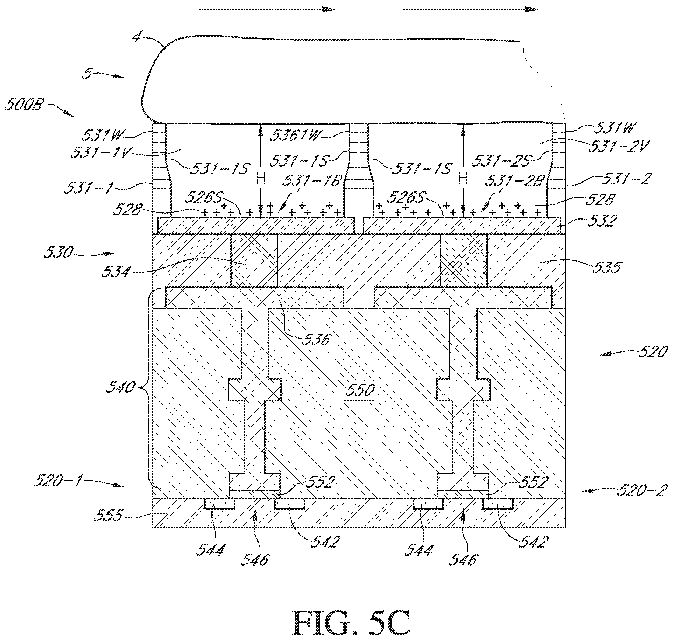

With respect to cell footprint on a sensor array of the present disclosure, FIG. 5B and FIG. 5C are schematic section views of a cell positioned on a sensor chip, such as sensor chip 520 of FIG. 2, which can be mounted in a sensor chip device, such as sensor chip device 500A of FIG. 5B and sensor chip device 500B of FIG. 5C. FIG. 5B and FIG. 5C illustrate generally structural components of a ChemFET sensor used for cell analysis. As will be described in more detail herein, FIG. 5B depicts cell 5 proximal to sensing surface 526S, while if FIG. 5C, cell 5 is plated upon microwells, such as microwell 531-1 and 531-2 of FIG. 5C.

In FIG. 5B and FIG. 5C, sensor chip 520 is depicted with first sensor 520-1 and second sensor 520-2. In various sensor array devices of the present disclosure, sensor chip 520 can include floating gate upper portion 530 coupled to sensor floating gate structure 540. Alternatively, various sensor array devices of the present disclosure, sensor chip 520 can include sensor floating gate structure 540. As depicted in FIG. 5B and FIG. 5C, floating gate upper portion 530 can include a top metal layer, sensor plate 532, as well as metal via 534, and floating gate metal layer 536 formed in dielectric 535. Metal layers 532, 534, and 136 can be a suitable metallic material, alloy, or conductive ceramic thereof, for example, titanium, silver, gold, platinum, and tungsten. Dielectric substrate 535 can be a dielectric material, such as silicon dioxide or silicon nitride.

As presented in the illustrated examples of FIG. 5B and FIG. 5C, sensor floating gate structure 540 can have metal layer 536 coupled to sensor plate 532 through metal via 534. Metal layer 536 is the uppermost floating gate conductor in sensor floating gate structure 540. As depicted in FIG. 5B and FIG. 5C, sensor floating gate structure 540 includes multiple layers of conductive material within layers of dielectric material 550. Sensors 520-1 and 520-2 can include conduction terminals including source/drain region 542 and source/drain region 544 within semiconductor substrate 555. Source/drain region 542 and source/drain region 544 comprise doped semiconductor material having a conductivity of a type different from the conductivity type of substrate 555. For example, source/drain region 542 and source/drain region 544 can comprise doped P-type semiconductor material, and substrate 555 can comprise doped N-type semiconductor material. Channel region 552 separates source/drain region 542 and source/drain region 544. Floating gate structure 540 overlies channel region 546, and is separated from substrate 555 by gate dielectric 552. Gate dielectric 552 can be silicon dioxide, for example. Alternatively, other suitable dielectrics can be used for gate dielectric 552 such as, for example materials with higher dielectric constants, silicon carbide (SiC), silicon nitride ($Si_3N_4$), silicon oxynitride ($Si_2N_2O$), aluminum nitride (AIN), hafnium dioxide ($HfO_2$), tin oxide ($SnO_2$), cesium oxide ($CeO_2$), titanium oxide ($TiO_2$), tungsten oxide ($WO_3$), aluminum oxide ($Al_2O_3$), lanthanum oxide ($La_2O_3$), gadolinium oxide ($Gd_2O_3$), and any combination thereof.

An end user can run applications using cell analysis systems of the present disclosure that controllably flow various reagents and solutions over the surface of sensor chip 520, as indicated by the arrows at the top of FIG. 5B and FIG. 5C. The temperature of the various reagents and solutions can be controlled to ensure that they match a desired temperature set by an end user. As such, a cell analysis system of the present disclosure can maintain solutions and reagents at an end user selected temperature of between about 10 °C to about 50 °C; moreover a selected temperature in a range of between about 20 °C to about 37 °C, which is a range in which many cell-based assays are conducted.

Additionally, various gases, such as oxygen and carbon dioxide, can be selected for equilibration with various reagents and solutions, so that a desirable partial pressure of a gas or gases can preferentially provide a desired amount of dissolved gases in a reagent or solution. For example, cells growing in bicarbonate buffered cell culture medium require a compressed air composition that includes 5% carbon dioxide. Alternatively, for studies of cell hypoxia, ambient oxygen levels in the gas source can be varied from normal levels of 20% oxygen down to 1%.

Table 1 summarizes attributes of exemplary ChemFET sensor array devices of the present disclosure (Chip Device 1 is not in accordance with the present invention, Chip Devices 2-4 are in accordance with the present invention):

Table 1: Exemplary sensor array devices

| Chip Device | Pixels per Device | Pitch ($\mu$m) | Pixel Size ($\mu$m x $\mu$m) | Pixel Area $\mu m^2$/pixel | $FR_{max}$ per Device (fps) | $FR_{max}$ per Row (fps) |
|---|---|---|---|---|---|---|
| 1 | 40M | 3.36 | 2.910 x 3.360 | 9.78 | 120 | 75,000 |
| 2 | 165M | 1.68 | 1.453 x 1.680 | 2.44 | 30 | 75,000 |
| 3 | 300M | 1.26 | 1.093 x 1.262 | 1.38 | 30 | 162,000 |
| 4 | 660M | 0.85 | 0.733 x 0.846 | 0.62 | 15 | 162,000 |

A sensor array device of the present disclosure can have between about 40M to about 660M pixels, with a center-to-center spacing between each sensor; or the pitch. The pitch of sensor array devices according to the present invention is between 850 nm to about 3.3 $\mu$m. With respect to data collection, a collection of sensor output signals from all sensors in an array constitutes a frame of data. For various sensor array devices of the present disclosure with between about 20 million pixels to about 660 million pixels, a frame of is a data file of substantial size, which is collected in units of hertz (Hz) as frames per second. Further, there is an inverse relationship between an area of interest selected representing the number of pixels and the rate at which data can be collected, so that by selecting a smaller subset of pixels to monitor, i.e. by windowing down the area of a sensor array device over which data is collected, frame rate can be increased.

The impact of windowing down is evidenced in Table I by comparison of the values entered in the second to last column, which is maximum frame rate for collecting data from an entire device, to the values entered into the last column, which is maximum frame rate for collecting data from a single row of a device. As such, by windowing down to collect data from a single row, frame rate is substantially increased. According to the present disclosure, various sensor array devices of the present teaching can have a device frame rate for outputting data from an array of between about 15 fps to about 240 fps. In contrast, the frame rate per row is between about 75, 000 to about 162,000 for a single row of an array of the chip devices of Table 1, serving to emphasize the impact of windowing down an area of interest.

As will be described in more detail herein, as depicted in FIG. 5B and FIG. 5C, sensing surface 526S of sensor plate 532 can act as the sensor surface for monitoring changes in, for example, cell electrophysiology and metabolism for cells subjected to any of a variety of conditions or stimuli. In that regard, cell 5 shown in FIG. 5B and FIG. 5C as a partial section of a cell, is depicted as positioned over sensor plate 532 of sensors 520-1 and 520-2.

In FIG. 5B, cell 5 is depicted as plated on surface coating 524, which has been applied to sensing surface 526S of sensor chip 520. In FIG. 5C, cell 5 is depicted as plated on microwells 531-1 and 531-2 of sensor chip 520. Though not depicted in FIG. 5C, a chip device with microwells, such as sensor chip 500B of FIG. 5C, can also be coated with a biocompatible coating. Accordingly, as will be described in more detail herein, a surface coating applied to either a sensor chip device with or without wells can be any cell-compatible material, such as various biopolymer materials including poly-D-lysine, laminin, fibronectin, collagen, and combinations thereof, as well as various preparations of extracellular matrix (ECM).

Regarding FIG. 5C, which depicts sensor chip 520 of sensor device 500B with microwells 531-1 and 531-2 formed over sensor 520-1 and sensor 520-2, respectively, such microwells are exemplary of an array of microwells that can be formed over a sensor array, for example, over a sensor array for any of the sensor array devices of Table 1. As depicted in FIG. 5C, each microwell is capacitively coupled to at least one sensor. In that regard, though FIG. 5C depicts one microwell per sensor, various sensor array devices of the present teachings can have, for example, between 1-4 sensors capacitively coupled to a microwell. Exemplary microwells 531-1 and 531-2 of FIG. 5C are containment structures formed by microwell walls 531W, a side of each wall defining sidewalls 531-1S and 531-2S, respectively, while the top surface of a sensor 531-1B and 531-2B, respectively can provide the bottom of the microwell structure. According to the present disclosure, microwells, such as exemplary microwells 531-1 and 531-2 of FIG. 5C, are containment structures that define a microwell volume, such as microwell volume 531-1V and microwell 531-2V of FIG. 5C. Microwells, such as exemplary microwells 531-1 and 531-2, can prevent various chemical analytes from diffusing away from the sensor, for example, analytes of interest released by a cell proximal to a sensor.

In that regard, chip devices with wells or no wells can be selected by an end user according to what analysis is being performed. For example, as previously described herein, a cell is capacitively coupled to the sensor array surface, and accordingly, ChemFET-based cell analysis systems of the present disclosure can measure changes in cell membrane potential. Given that the measured change of cell membrane potential drops as the square of the distance of a cell from a sensor surface, a chip device without wells, such as chip device 500A of FIG. 5B, can be selected by an end user, thereby allowing cells to adhere to a prepared surface of a chip device. As previously described herein, microwells, such as exemplary microwells 531-1 and 531-2 of FIG. 5C, can prevent various chemical analytes from diffusing away from the sensor. As such, an end user can select a chip device with microwells, such as chip device 500B of FIG. 5C, when the detection of uptake or release of chemical analytes from a cell is the target of a study.

With respect to the bottom width of a microwell, such as microwell bottom surface 531-1B and 531-2B of FIG. 5C, the width of a microwell at the bottom of the microwell over a sensor plate should be about 90% of the width of sensor plate 532. As summarized in Table 1, for various types of sensor array devices, the center-to-center spacing between each sensor; or the pitch, is between about 850 nm to about 3.3 $\mu$m, while a sensor plate, such as sensor plate 532 of FIG. 5C, can have a width of between about 600 nm to about 3.1 $\mu$. By way of a non-limiting example, for a sensor plate, such as sensor plate 532 of FIG. 5C, with a width of about 600 nm, various microwell arrays can have a width of about 540 nm the bottom of the microwell over the sensor plate, while for a sensor plate with a width of 3.1 $\mu$, various microwell arrays can have a width of about 2.8 $\mu$ the bottom of the microwell over the sensor plate.

With respect to the width-to-height aspect ratio of microwells, for various microwell arrays, the width-to-height ratio of a microwell can be between about 1:2 to about 1:4. As depicted in FIG. 5C, microwells 531-1 and 531-2 have bottom width 531-1B and 531-2B, respectively, and height H. By way of a non-limiting example, for various microwell arrays that have microwells with a width of about 540 nm, the microwell height, H, can be up to about 1.1 $\mu$, while for various microwell arrays that have microwells with a width of about 2.8 $\mu$, the microwell height can up to about 5.6 $\mu$.

As previously described herein, floating gate upper portion 530 of FIG.5C can include a top metal layer, sensor plate 532, as well as metal via 534 and metal layer 536, all of which can be formed in dielectric substrate 535. Sensing surface 526S can be formed as a top layer over various the surfaces that define each microwell of a microwell array. For example, sensing surface 526S can be formed over sensor plate 532. Alternatively, sensing layer 526S can be formed as a continuous layer over sensor plate 532 and partially or completely over the sidewalls of a microwell, such as sidewalls 531-1S and 531-2S of microwells 531-1 and 531-2, respectively of FIG. 5C. Sensing layer 526S for both chip device sensor chip 520 of FIG. 5B and chip sensor chip 520 of FIG. 5C can be formed as a metal oxide or metal nitride layer, such as titanium oxide, titanium nitride, and titanium oxynitride. According to the present disclosure, ChemFETs that can be fabricated with sensing surfaces modified to be selective for the analysis of a targeted chemical species of interest for cell biology, for example, such as glucose, sucrose, lactate and urea. By way of another non-limiting example, ion sensitive field-effect transistors (ISFETs) can have sensing surfaces modified to be selective for various ions of interest; particularly for various cell metabolism studies, such as hydrogen, potassium, calcium and chloride. As will be described in more detail herein, ISFET sensor arrays selective for hydrogen, with the attributes of cells as provided in Table 1, can be used to detect any change in the state of a cell that can change the surface potential of such an ISFET sensor.

Sensors 520-1 and 520-2 of FIG. 5B and FIG. 5C are responsive to changes in the surface potential of ion layer 528 proximate to sensing surface 526S, which can cause changes in the voltage on floating gate 540. As such, an applied reference voltage, as previously described herein for FIG. 2A, ensures that the voltage of the floating gate exceeds a threshold voltage, providing that small changes in the floating gate voltage can cause current to flow through channel region 546, resulting in an output signal for sensors 520-1 and 520-2. In that regard, changes to the surface potential of ion layer 528 can be measured by measuring the current in channel region 546 between, for example, source region 542 and drain region 544. As such, sensors 520-1 and 520-2 of FIG. 5B and FIG. 5C can be used directly to provide a current-based output signal on an array line connected to source region 542 or drain region 544, or indirectly with additional circuitry to provide a voltage-based output signal.

As such, any change in the state of cell 5 of FIG. 5B and FIG. 5C that can alter the surface potential in ion layer 528 can be monitored by various ChemFET sensor array devices of the present disclosure, such as chip device 500A of FIG. 5B, and chip device 500B of FIG. 5C. With respect to output signal, any cell activity that can increase surface potential would result in an output signal of positive amplitude for a ChemFET sensor, while any cell activity that can decrease surface potential would result in an output signal of negative amplitude for a ChemFET sensor. In that regard, any change in the state of a cell that can change the surface potential of a ChemFET sensor can result in a measurable output signal. For example, when using an ISFET sensor, any metabolic activity that can increase the concentration of a cationic species for which an ISFET sensor is selective for would cause an increase in surface potential. The result would be an output signal of positive amplitude for that ISFET sensor. Conversely, any metabolic activity that can decrease the concentration of a cationic species for which an ISFET sensor is selective for would cause an decrease in surface potential. The result would be an output signal of negative amplitude for that ISFET sensor. In another example, the surface potential can be altered by the capacitive coupling of cell 5 to sensor chip 520 of FIG. 5B and FIG. 5C, respectively, so that as the electrical potential across cell membrane 4 changes in response to a chemical or electrical stimulus. Such a change electrical potential across the cell membrane can be detected by ChemFET sensors 520-1 and 520-2 of FIG. 5B and FIG. 5C.

Data collected in experiments monitoring cellular response to various stimuli with various embodiments of ChemFET sensor array devices of the present teachings can be presented to an end user in a number of formats. In one format, temporal response is presented as a graph of detector counts that can be readily correlated to millivolt (mV) or microvolt (μV) changes in sensing surface potential as a function of time. With respect to electroscopic imaging, as the response for each sensor is continuously detected, a spatial image of detector response can be displayed to an end user associated with a footprint of a cell. For example, for any of a selected time over a course of a selected application, a spatial visualization of cells can be presented as an electroscopic image. Further, an electroscopic image video can be presented for the dynamic visualization of cells over a selected part or over the entire course of an experiment.

Accordingly, for various cell analysis systems of the present disclosure, an end user can select a sensor array device from a variety of sensor array devices having different attributes that that can be matched to a cell analysis of interest. For example, a sensor array device used for the detection of the electrophysiological activity of a cell can have different attributes than a sensor array device used for the detection of specific analytes for a metabolic study.

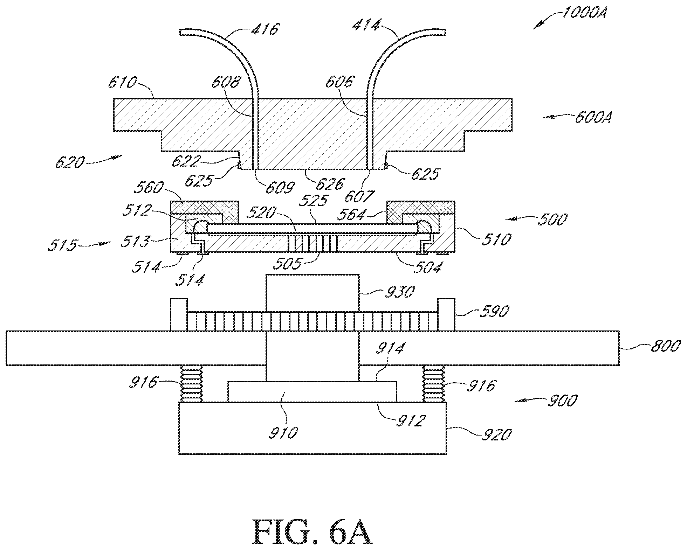

FIG. 6A is a schematic representation that illustrates generally chip clamp assembly 1000A of a cell analysis system of the present disclosure. It should be noted that the schematic representation of chip clamp assembly 1000A does not represent the relative scale of the components, moreover their relationship and function. As depicted in FIG. 6A, chip clamp assembly 1000A is an apparatus that includes chip interface device 600A, which includes fluidic interface 610 and chip interface device flange 620. Fluidic interface 610 can include chip interface device inlet channel 606, which is fabricated in chip interface device 600A and terminates as chip interface device inlet port 607 on chip device interface bottom surface 626. As illustrated in FIG. 6A, is in fluid communication with input line 414, and can be placed into fluid communication with any of fluidic manifold input lines 414A-414E as depicted in FIG. 1A. Similarly, chip outlet channel 608 is fabricated in chip interface device 600A and terminates as chip interface device outlet port 609 on chip device interface bottom surface 626. As illustrated in FIG. 6A, chip interface device outlet channel 608 is in fluid communication with output line 416, and can be placed into fluid communication with any of fluidic manifold output lines 416A-416E as depicted in FIG. 1A. Though shown as being directly across from one another in the schematic illustration of FIG. 6A, chip interface device inlet port 607 and chip interface device outlet port 609 can be located diagonally, so that fluid flows into one corner of a chip device and flows out of an opposite diagonal corner of the chip device.

Chip interface device flange 620 can include flange rim 622, flange rim O-ring 625, and chip device interface bottom surface 626. Chip interface device flange 620 can engage with and reversibly couple to fluid dam 560, so that flange rim O-ring 625 is seated against the sealing surface of fluid dam inner wall surface 564 to provide a reversable seal between chip interface device flange 620 of chip interface device 600A and a chip device 500. Once reversibly sealed, chip device interface bottom surface 626 is functionally a flow cell cover that defines a flow cell having a flow cell chamber, or analysis chamber, with a defined height.

Recalling for FIG. 2, chip device substrate 510 includes a first set 515A and a second set 515B of electronic connections, each of which can comprise two rows of interconnections between sensor chip 520 and a pogo pin block. As depicted in FIG. 6A, each connection in a set of electronic connections 515 can include a bond wire, such as bond wire 512, connected to sensor chip 520. Bond wire 512 is connected to pad 514 through chip circuit 513, which includes lines and vias, and is set in encapsulant 513. Pad 514 connects bond wire 512 to a pogo pin block. As depicted in FIG. 6A, sensor array device 500 can be connected to pogo pin block 590. As previously described herein, chip clamp assembly 1000 of FIG. 1A, is an apparatus that can provide a clamping force to chip interface device 600A to engage chip device 500 with pogo pin block 590. In turn pogo pin block 590 is engaged with a PC board mounted to chip clamp base 800. The integrated circuit connections provide power, timing and control to, as well as data acquisition from a sensor chip by an cell analysis system.

Accordingly, several layers of electronic interconnections are involved in the function of chip device 500, all in proximity to a flow cell fluidically connected to a fluidic system, such as fluidic system 400 of FIG. 1A. Moreover, sensor array device 500 is an open structure for the purpose of providing access to an end user for plating and manipulations of cells, as well as being a component that must be easily removable and replaceable from an analysis platform of a cell analysis system. As such, various chip interface devices of the present disclosure are configured to provide consistent leak-free engagement with a sensor device, such as sensor array device 500 of FIG. 2, as well as providing leak-free repetitive engagement of various chip interface devices of the present disclosure with a sensor device.

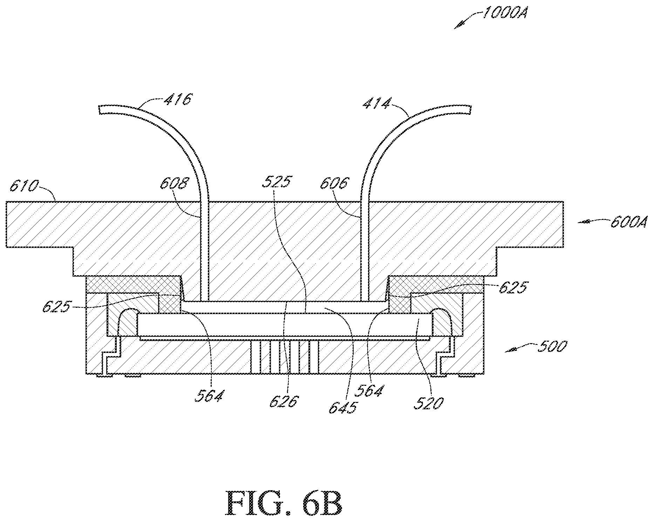

FIG. 6B is a schematic representation that illustrates generally flow cell 645 formed between chip interface device 600A and chip device 500. It should be noted that the schematic representation of flow cell 645 formed between chip interface device 600A and chip device 500 does not represent the relative scale of the components, moreover their relationship and function. As previously described herein, chip interface device 600A includes fluidic interface 610 and chip interface device flange 620 (FIG. 6A) with flange rim O-ring 625. Fluidic interface 610 can include chip interface device inlet channel 606, which is fabricated in chip interface device 600A and terminates as chip interface device inlet port 607 (FIG. 6A) on chip device interface bottom surface 626. Chip interface device inlet channel 606 is in fluid communication with input line 414, and can be placed into fluid communication with any of fluidic manifold input lines 414A-414E as depicted in FIG. 1A. Similarly, chip outlet channel 608 is fabricated in chip interface device 600A and terminates as chip interface device outlet port 609 (FIG. 6A) on chip device interface bottom surface 626. Chip interface device outlet channel 608 is in fluid communication with output line 416, and can be placed into fluid communication with any of fluidic manifold output lines 416A-416E as depicted in FIG. 1A.

As previously described herein, and as depicted in FIG. 6B, chip interface device flange 620 can engage with and reversibly couple to fluid dam 560 (FIG. 6A), so that flange rim O-ring 625 is seated against the sealing surface of fluid dam inner wall surface 564 to provide a reversable seal between chip interface device flange 620 (FIG. 6A) of chip interface device 600A and a chip device 500. Once reversibly sealed, flow cell 645 is formed between sensor chip top surface 525 and chip device interface bottom surface 626. Flow cell 645 is operably an analysis chamber with a defined height. As will be described in more detail herein, the defined height can be between 30 $\mu$m to about 770 $\mu$m, providing a flow cell volume of between 10 $\mu$l to about 310 $\mu$l.

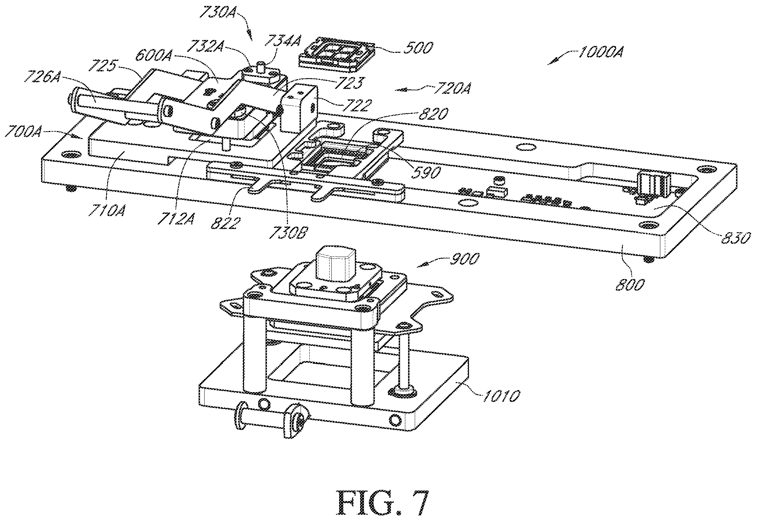

FIG. 7 is an exploded perspective view that illustrates generally chip clamp assembly 1000A. As depicted in FIG. 7, chip interface device shuttle 700A includes chip interface mounting frame 710A with chip interface mounting frame, upon which chip interface device 600A is mounted. Chip interface mounting frame 710A includes aperture 712A, and chip interface device clamping assembly 720A. As depicted in FIG. 7, chip interface device clamping assembly 720A can include lever arm 723, which is engaged with clamping arm fulcrum 722. As depicted in FIG. 7, lever arm 723 and lever arm 725 are joined by lever arm handle 726A at the ends opposite where they are joined to a fulcrum. Chip interface device 600A can be mounted to chip interface device shuttle 700A by mounting to lever arm 723. Chip interface device shuttle 700A is mounted to a linear positioning system (not shown), which is mounted in chip interface device shuttle linear positioning system run (not shown).

Once interface device 600A is moved into position over sensor array device 500, chip interface device 600A can be engaged for use by applying a downward movement of lever arm 723 pivoting on fulcrum 722. The downward movement applies a vertical force with respect to two linear bearings 730a and 730b, each bearing including a bushing, such as bushing 732a of linear bearing 730a, that rides on a vertical shaft that is mounted to chip interface mounting frame 710A, such as vertical shaft 734a of linear bearing 730a. In order to fully engage the chip with the chip clamp assembly, a clamping force of between about 7 lbs. to about 14 lbs. can be applied to lever arm 723. A clamping force of between about 7 lbs. to about 14 lbs. applied to lever arm 723 translates into a load force of between about 30 lbs. to about 60 lbs. +/- 1 lbs. applied to chip interface device 600A.

Chip clamp base 800 can include chip device mount 820 for mounting chip device 500. As depicted in FIG. 7, pogo pin block 590 is mounted in chip device mount 820. Chip device 500 can mounted into chip device mount 820, and upon pogo pin block 590. Chip device mount scissor clamp 822 can be used to securely hold chip device 500 in chip device mount 820. Pogo pin block 590 is electronically connected to printed circuit (PC) board 830, mounted to chip clamp base 800. Chip clamp assembly 1000A is an apparatus that can be mounted to chip clamp assembly mounting plate 1010.

At initial use, chip device 500 can be mounted upon pogo pin block 590 in chip device mount 820, and secured into place with chip device mount scissor clamp 822. Chip interface device shuttle 700A can be positioned over chip device 500 using the linear positioning system. Using chip interface device clamping assembly 720A, a clamping force of between about 7 lbs. to about 14 lbs. can be applied to lever arm 723, thereby applying a load force of between about 30 lbs. to about 60 lbs. +/- 1 lbs. to chip interface device 600A, so that chip interface device 600A can be engaged to provide contact between chip device 500 and pogo pin block 590, as well as ensuring contact of a TEC with a TEC thermal control assembly, such as ensuring contact of TEC 910 with TEC cooling assembly 920 of FIG. 6A. In addition to providing engagement with electrical and thermal control components with respective devices and assemblies, application of a clamping force also provides that chip interface device 600A can be positioned to be sealably engaged with and reversibly coupled to sensor array device 500, thereby forming a flow cell, such as flow cell 645 of FIG. 6B, which is operatively also an analysis chamber.

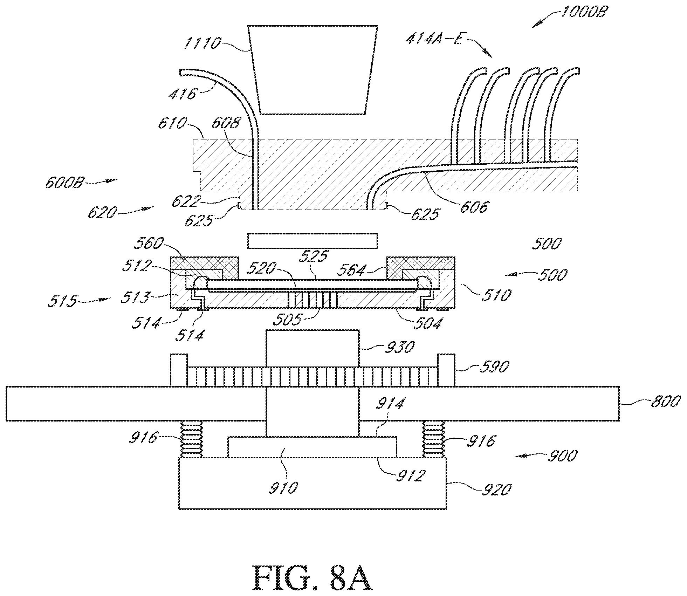

FIG. 8A is a schematic representation that illustrates generally chip clamp assembly 1000B of a cell analysis system of the present disclosure. As noted for FIG. 6A, the schematic representation of chip clamp assembly 1000B does not represent the relative scale of the components of the apparatus, moreover their relationship and function. As depicted in FIG. 8A, microscope objective 1110, which can be part of a microscope assembly, such as microscope assembly 1100 of FIG. 1A, is positioned relative to flow cell cover 640 of optical interface 630 of chip clamp assembly 1000B. As will be described in more detail subsequently, in comparison to chip interface device 600A of FIG. 6A that includes a fluidic and chip device interface component, chip interface device 600B additionally includes optical interface 630.

In FIG. 8A, chip interface device 600B is presented with a hatched perimeter line to connote that fluidic interface 610 for chip interface device 600B is not in the same plane as the optical interface 630 of chip interface device 600B. As indicated in FIG. 8A, and in comparison to chip interface device 600A of FIG. 6A, in order to provide optical access to a chip sensor device, the fluidic component of chip interface device 600B is adjacent to optical interface 630 in chip interface device 600B. During an analysis, flow cell cover 640, with flow cell cover upper surface 641 and flow cell cover lower surface 643, is positioned over chip device 500, so that flow cell cover lower surface 643 defines the top surface of a flow cell. Flow cell cover 640 is an optically transparent material, such as a borosilicate glass, and therefore provides for microscopic viewing of sensor chip top surface 525, upon which cells can be plated.

As depicted in FIG. 8A, in addition to optical interface 630, chip interface device 600B additionally includes fluidic interface 610 and chip interface device flange 620. Fluidic interface 610 can include chip interface device inlet channel 606, which is fabricated in chip interface device 600B and terminates as chip interface device inlet port 607 at the bottom of flange rim 622. As illustrated in FIG. 8A, chip interface device inlet channel 606 can be placed into fluid communication with any of fluidic manifold input lines 414A-414E (see FIG. 1A). Similarly, chip outlet channel 608 is fabricated in chip interface device 600B through chip interface device flange 620 and terminates as chip interface device outlet port 609 at the bottom of flange rim 622. As illustrated in FIG. 8A, chip interface device outlet channel 608 can be placed into fluid communication with any of fluidic manifold waste lines 416A-416B (see FIG. 1A). Though shown as being directly across from one another in the schematic illustration of FIG. 8A, chip interface device inlet port 607 and chip interface device outlet port 609 can be located diagonally, so that fluid flows into one corner of a chip device and flows out of an opposite diagonal corner of the chip device.

Chip interface device flange 620 can engage with and reversibly couple to fluid dam 560, so that flange rim O-ring 625 is seated against the sealing surface of fluid dam inner wall surface 564 to provide a reversable seal between chip interface device flange 620 of chip interface device 600B and a chip device 500. Once reversibly sealed, optically transparent flow cell top cover 640 is functionally a flow cell cover that defines a flow cell that is an analysis chamber with a defined height.

In addition to fluidic control of fluid flow through chip device 500, precision thermal control of a sensor array device 500 is provided for various cell analysis systems of the present disclosure. Accordingly, for both FIG. 6A and FIG. 8A, chip thermal control assembly 900 is shown with thermoelectric cooling (TEC) device 910 mounted on cooling assembly 920, with first TEC surface 912 in contact with TEC cooling assembly 920. Second surface 914 of TEC device 910 is in contact with chip device cooling block 930, which during use of a chip device is in contact with device substrate lower surface 504. Thermal vias 505 in chip device substrate 510 provide effective thermal contact between chip device cooling block 930, chip device substrate 510 and sensor chip 520. TEC cooling assembly 920 is mounted to the bottom of chip clamp base 800 with springs 916 to exert sufficient force on chip thermal control assembly 900 to make effective thermal contact with chip device 500. The force exerted by springs 916 on chip thermal control assembly 900 is about 4 lbs. +/- 10%. Chip thermal control assembly 900 can provide precision thermostatting of a chip device, such as chip device exemplified in Table 1, over a temperature range of 4 °C to 60 °C within +/- 0.1 C.

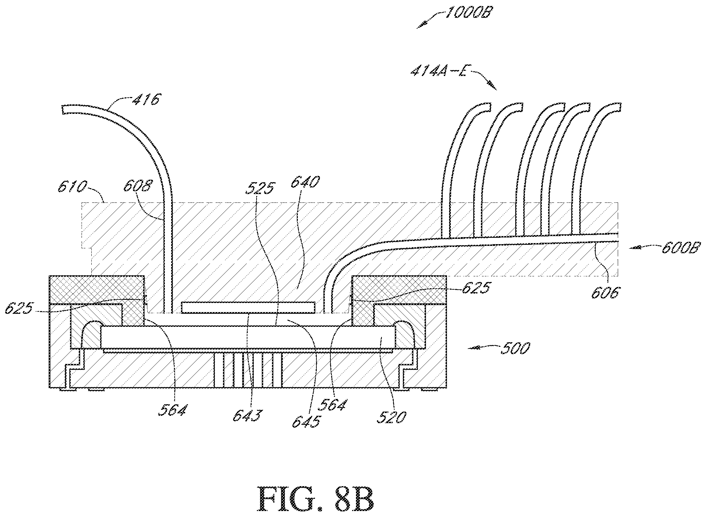

FIG. 8B is a schematic representation that illustrates generally flow cell 645 formed between chip interface device 600B and chip device 500. It should be noted that the schematic representation of flow cell 645 formed between chip interface device 600B and chip device 500 does not represent the relative scale of the components, moreover their relationship and function.

As previously described herein, chip interface device 600B is presented with a hatched perimeter line to connote that fluidic interface 610 for chip interface device 600B is not in the same plane as an optical interface that includes optically transparent flow cell cover 640 with flow cell cover upper surface 641 (FIG. 8A) and flow cell cover lower surface 643. Chip interface device 600B includes fluidic interface 610 and chip interface device flange 620 (FIG. 8A) with flange rim O-ring 625. Fluidic interface 610 can include chip interface device inlet channel 606, which is fabricated in chip interface device 600B and terminates as chip interface device inlet port 607 (FIG. 8A) on chip device interface bottom surface 626. Chip interface device inlet channel 606 can be placed in fluid communication with with any of fluidic manifold input lines 414A-414E as depicted in FIG. 1A. Similarly, chip outlet channel 608 is fabricated in chip interface device 600B and terminates as chip interface device outlet port 609 (FIG. 8A) on chip device interface bottom surface 626. Chip interface device outlet channel 608 is in fluid communication with outlet line 416, which can be placed in fluid communication with any of fluidic manifold output lines 416A-416E as depicted in FIG. 1A.

As previously described herein, and as depicted in FIG. 8B, chip interface device flange 620 can engage with and reversibly couple to fluid dam 560 (FIG. 8A), so that flange rim O-ring 625 is seated against the sealing surface of fluid dam inner wall surface 564 to provide a reversable seal between chip interface device flange 620 (FIG. 8A) of chip interface device 600A and a chip device 500. Once reversibly sealed, flow cell 645 is formed between sensor chip top surface 525 and flow cell cover lower surface 643. Flow cell 645 is operably an analysis chamber with a defined height. As will be described in more detail herein, the defined height can be between 30 $\mu$m to about 770 $\mu$m, providing a flow cell volume of between 10 $\mu$l to about 310 $\mu$l.

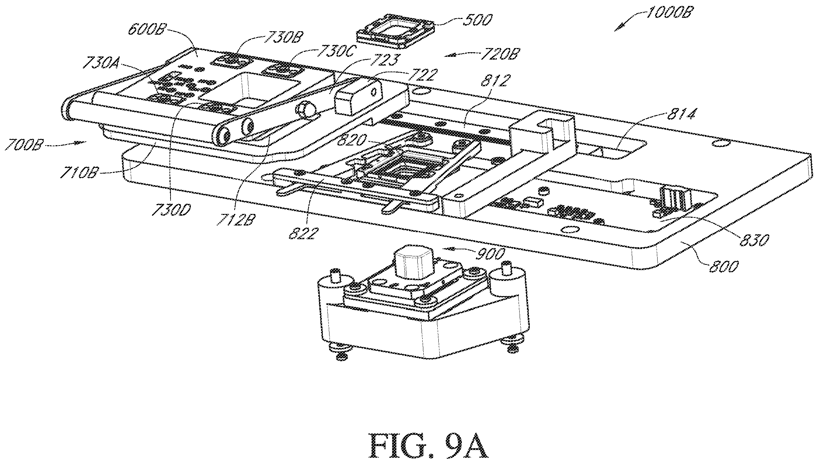

FIG. 9A is an exploded perspective view that illustrates generally chip clamp assembly 1000B. As depicted in FIG. 9A, many of the feature of chip clamp assembly 1000B are similar to those of, for example, chip clamp assembly 1000A of FIG. 7.

As depicted in FIG. 9A, chip interface device shuttle 700B includes chip interface device mounting frame 710B, upon which chip interface device 600B is mounted. Chip interface device mounting frame 710B includes chip interface device mounting frame aperture 712B, and chip interface device clamping assembly 720B. As depicted in FIG. 9A, chip interface device clamping assembly 720B can include lever arm 723, which is engaged with chip device interface clamping arm fulcrum 722. As depicted in FIG. 9A, lever arm 723 and lever arm 725 are joined by lever arm handle assembly 726B at the ends opposite where they are joined to a fulcrum. Chip interface device shuttle 700B is mounted to chip interface device shuttle linear positioning system 812, which can be mounted in chip interface device shuttle linear positioning system run 814.

Once interface device 600B is moved into position over sensor array device 500, chip interface device 600B can be engaged for use by applying a downward movement of lever arm 723 pivoting on fulcrum 724. As interface device 600B is larger than interface device 600A of FIG. 7, four linear bearings 730a-730d are used to provide even vertical force on interface device 600B. The downward movement applies a vertical force with respect to linear bearings 730-730d, each bearing including a bushing that rides on a vertical shaft that is mounted to a chip interface mounting frame, such as is shown and described for FIG. 7. To fully engage the chip with the chip clamp assembly, a clamping force of between about 7 lbs. to about 14 lbs. can be applied to lever arm 723. A clamping force of between about 7 lbs. to about 14 lbs. applied to lever arm 723 translates into a load force of between about 30 lbs. to about 60 lbs. +/- 1 lbs. applied to chip interface device 600B.

Chip clamp base 800 can include chip device mount 820 for mounting chip device 500. As depicted in FIG. 9A, pogo pin block 590 is mounted in chip device mount 820. Chip device 500 can mounted into chip device mount 820, and upon pogo pin block 590. Chip device mount scissor clamp 822 can be used to securely hold chip device 500 in chip device mount 820. Pogo pin block 590 is electronically connected to printed circuit (PC) board 830, mounted to chip clamp base 800. Though chip clamp assembly 1000A is depicted in FIG. 7 is an apparatus mounted to chip clamp assembly mounting plate 1010, as will be described in more detail herein, chip clamp assembly 1000B is an apparatus that can be mounted to an X,Y positioning system (not shown).

At initial use, chip device 500 can be mounted upon pogo pin block 590 in chip device mount 820, and secured into place with chip device mount scissor clamp 822. Chip interface device shuttle 700B can be positioned over chip device 500 using chip interface device shuttle linear positioning system 812, so that optical interface compartment 630 is positioned over chip device 500. As previously described herein, using chip interface device clamping assembly 720B, a clamping force to a clamping force of between about 7 lbs. to about 14 lbs. can be applied to lever arm 723, thereby applying a load force of between about 30 lbs. to about 60 lbs. +/- 1 lbs. to chip interface device 600B, so that chip interface device 600B can be engaged to provide contact between chip device 500 and pogo pin block 590, as well as ensuring contact of a TEC with a TEC thermal control assembly, such as ensuring contact of TEC 910 with TEC cooling assembly 920 of FIG. 8A. In addition to providing engagement with electrical and thermal control components with respective devices and assemblies, application of a clamping force also provides that chip interface device 600B can be positioned to be sealably engaged with and reversibly coupled to sensor array device 500, thereby forming a flow cell, such as flow cell 645 of FIG. 8B. As depicted in FIG. 8B, flow cell 645 is formed between sensor chip top surface 525 and flow cell cover lower surface 643.

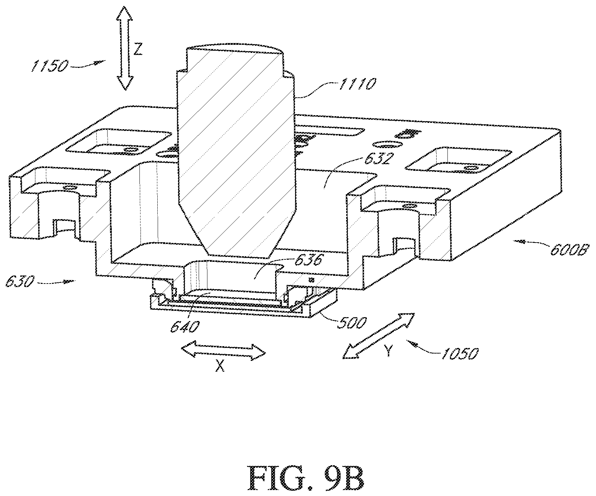

FIG. 9B is a perspective schematic section view that illustrates generally alignment of a microscope objective over a sensor array device for a cell analysis system of the present disclosure. Though chip clamp assembly 1000A is depicted in FIG. 7 is an apparatus mounted to chip clamp assembly mounting plate 1010, chip clamp assembly 1000B is an apparatus that can be mounted to an X,Y positioning system. As depicted in FIG. 9B, optical interface 630 of chip interface device 600B can include objective recess 632 and optical aperture 636. Microscope objective 1110 can focus on a sensor surface of chip device 500 through optically transparent flow cell cover 640 by being positioned over optical aperture 636. A chip clamp assembly can be mounted on chip clamp assembly positioning system 1050, indicated schematically in FIG. 9B. As previously described herein in reference to FIG. 9B, chip clamp assembly positioning system 1050 is an X,Y positioning system. Similarly, a microscope assembly, such as microscope assembly 1100 of FIG. 1A, can have Z-height positioning system 1150 for microscope objective 1110. In that regard, between the Z-height positioning of microscope objective 1110 and X,Y positioning of aperture 636 relative to microscope objective 1110, an end user can view cells over a sensor surface.

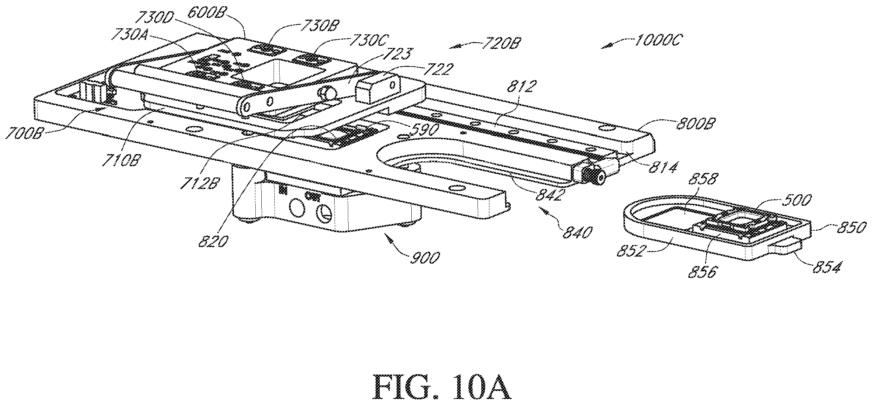

FIG. 10A is an exploded perspective view that illustrates generally chip clamp assembly 1000C. As depicted in FIG. 10A, chip clamp assembly 1000C has many of the features as previously described for chip clamp assembly 1000B of FIG. 9A.

For example, just as depicted in FIG. 9A, chip interface device shuttle 700B of FIG. 10A includes chip interface device mounting frame 710B, upon which chip interface device 600B is mounted. Chip interface device mounting frame 710B includes chip interface device mounting frame aperture 712B, and chip interface device clamping assembly 720B. Further, chip interface device clamping assembly 720B of FIG. 9A and FIG. 10A can include lever arm 723, which is engaged with chip device interface clamping arm fulcrum 722. Additionally, lever arm 723 and lever arm 725 of FIG. 9A and FIG. 10A are joined by lever arm handle assembly 726B at the ends opposite where they are joined to a fulcrum. Chip interface device shuttle 700B is mounted to chip interface device shuttle linear positioning system 812, which can be mounted in chip interface device shuttle linear positioning system run 814. As depicted in FIG. 9A and FIG. 10A, chip interface device shuttle 700B can be positioned over chip device mount 820, in which pogo pin block 590 is mounted. Additionally, as will be described in more detail herein, chip thermal control assembly 900 is depicted as mounted beneath chip claim assembly 1000B of FIG. 9A and chip claim assembly 1000C of FIG. 10A.

However, as depicted in FIG. 10A, chip clamp base 800B of chip clamp assembly 1000C is an apparatus that has chip tray dock 840 with chip tray dock track 842 for docking chip tray 850. Chip tray 850 has chip tray sidewall 852, which docks with chip dock 840. Additionally, as chip devices for cell analysis are open architecture, such as device 500 of FIG. 2, chip tray sidewall 842 provides for potential containment of fluids from a chip device prepared for cell analysis. Chip tray tab 854 provides for either manual or automated insertion of chip tray 850 into chip dock 840. Chip device 500 can be secured to chip tray mount 856, which, allows for pre-alignment of chip device 500 with chip interface device 600B mounted on chip interface device mounting frame 710B. Chip drying pad 858 of chip tray 850 can include bibulous sheet material, such as any laboratory grade bibulous paper that can provide blotting the bottom surface of chip device 500 before it is inserted into chip device mount 820.

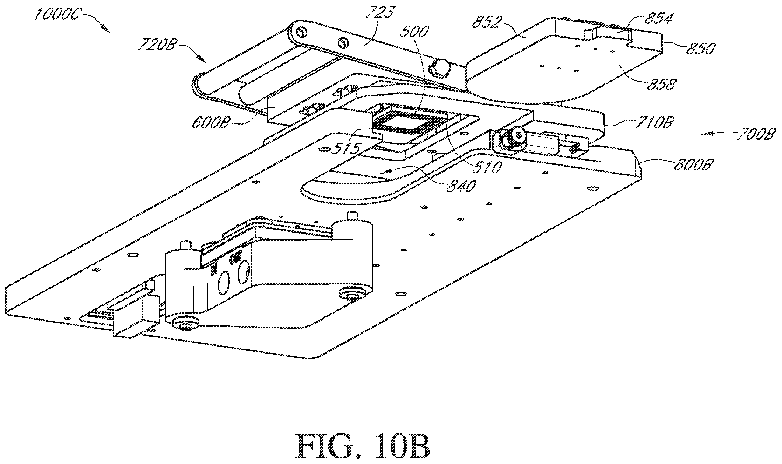

FIG. 10B is a bottom perspective view that illustrates generally chip clamp assembly 1000C, depicting chip device 500 attached to chip interface device 600B. Regarding the pre-alignment of chip device 500 by chip tray mount 856, chip interface device shuttle 700B can be positioned over a chip device secured to chip tray mount 856 when chip tray 850 is positioned in chip tray dock 840 of chip clamp base 800B. Using chip interface device clamping assembly 720B to apply a downward force on chip interface device 600B while it is positioned over chip device 500 on chip tray 500 docked in chip tray dock 840 results in chip device 500 engaged with and reversibly coupled to chip interface device 600B. In FIG. 10B, chip tray 850 is retracted to show chip device 500 attached to chip interface device 600B. The bottom perspective view of chip device 500 provides a view of device substrate 510 of chip device 500, as well as electronic connections 515 for engaging with a pogo pin block, such as pogo pin block 590 of FIG. 10A. To engage chip device 500 with chip interface device 600B, a clamping force of between about 7 lbs. to about 14 lbs. can be applied to lever arm 723 of clamping assembly 720B. A clamping force of between about 7 lbs. to about 14 lbs. applied to lever arm 723 translates into a load force of between about 30 lbs. to about 60 lbs. +/- 1 lbs. applied to chip interface device 600B.

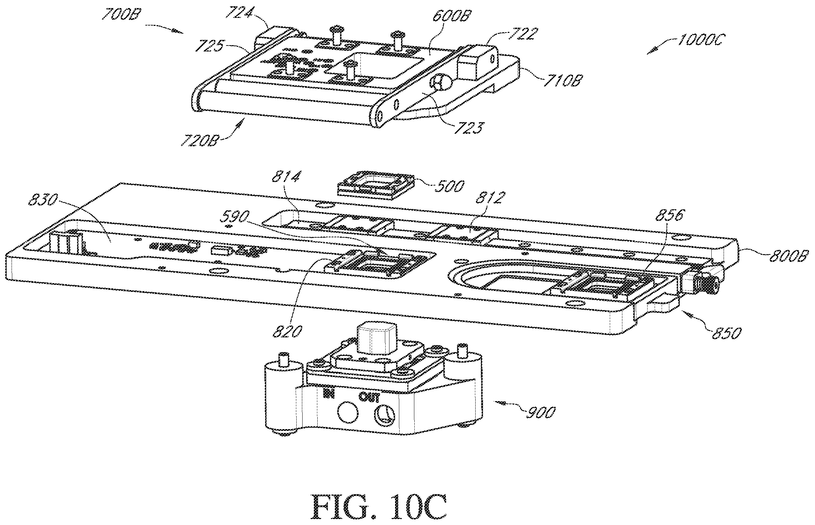

FIG. 10C is an exploded perspective view that illustrates generally chip clamp assembly 1000C with chip tray mount 856 of chip tray 850, which provides pre-alignment of a chip device for attachment to interface device 600B. Once chip device 500 is attached to interface device 600B, as depicted in FIG. 10B, chip interface device shuttle 700B can be moved to position sensor array device 500 over chip device mount 820 using chip interface device shuttle linear positioning system 812 mounted in chip interface device shuttle linear positioning system run 814 of chip clamp base 800B. Chip device 500 is shown in the exploded view of FIG. 10C in order to provide perspective regarding the position of a chip device attached to chip interface device 600B, as depicted in FIG. 10B with respect to chip device mount 820.

A chip device attached to a chip interface device shuttle can be engaged for use by applying a force on lever arms 723 and 724 pivoting on fulcrums 722 and 724, respectively. As previously described here for interface device 600B of FIG. 9A, four linear bearings 730a-730d are used to provide even vertical force on interface device 600B. The downward movement applies a vertical force with respect to linear bearings 730-730d, each bearing including a bushing that rides on a vertical shaft that is mounted to a chip interface mounting frame, such as is shown and described for FIG. 7. To fully engage the chip with the chip clamp assembly, a clamping force of between about 7 lbs. to about 14 lbs. can be applied to lever arm 723. A clamping force of between about 7 lbs. to about 14 lbs. applied to lever arm 723 translates into a load force of between about 30 lbs. to about 60 lbs. +/- 1 lbs. applied to chip interface device 600B. Once the force has been applied, chip device 500 is engaged with pogo pin block 590 mounted on chip device mount 820. Pogo pin block 590 is electronically connected to printed circuit (PC) board 830, mounted to chip clamp base 800B. As previously described herein, the clamping force also ensures proper contact of chip device 500 with a TEC, such as TEC 910 of FIG. 8A, which is part of chip thermal control assembly 900 of FIG. 10C.

Finally, though chip clamp base 800B is depicted with chip interface device 600B utilizing chip interface device shuttle 700B for clamping assembly 1000C of FIG. 10A-FIG. 10C, chip clamp base 800B can be used with chip interface device 600A utilizing chip interface device shuttle 700A, as depicted in for clamping assembly 1000A of FIG. 6A and FIG. 7.

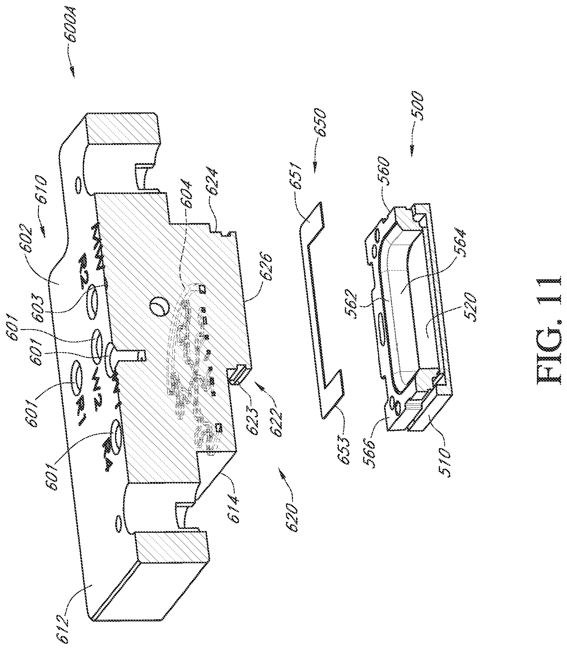

FIG. 11 is an exploded section view that illustrates generally chip interface device 600A with first surface 612 and second surface 614 of the present disclosure in relationship to chip device 500 as they would be aligned during an analysis. As previously described herein, chip interface device 600A can be integrated with clamping assembly 1000A as depicted in FIG. 6A and FIG. 7. Clamping assembly 1000A as depicted in FIG. 6A and FIG. 7 can be integrated with cell analysis system 2000 of FIG. 1A, which can have a fluidic system, such as fluidic system 400 of FIG. 1A and 1B.

As depicted in FIG. 11, fluidic interface 610 has fluidic interface block 602, which can be in fluid communication with microfluidic multiplexer circuit 604. In turn microfluidic multiplexer circuit 604 can be in fluid communication with a chip interface device inlet port and a chip interface device outlet port, such as chip interface device inlet port 607 of FIG. 6A. As shown in FIG. 6A, chip interface device inlet port 607 can be in fluid communication with any of fluidic manifold input lines 414A-414E through chip interface device inlet channel 606. Fluidic interface block 602 of FIG. 11 can have a fluidic interface block inlet port for each reagent or solution contained in a reagent or solution container of a fluidic system, such as solution and reagent containers 420A-420E, 430 and 440 of FIG. 1A. As depicted in FIG. 11, a plurality of fluidic interface block input line connectors 601 are shown, each of which can be connected to one of an input line, such as any of fluidic manifold input lines 414A-414E shown in FIG. 1A. In that regard, each fluidic system line is in fluid communication with a fluidic interface block inlet port, each of which in turn are in fluid communication with a microfluidic circuit branch of microfluidic multiplexer circuit 604. Each microfluidic circuit branch of microfluidic multiplexer circuit 604 can be selectively placed in fluid communication with a chip interface device inlet channel, such as chip interface device inlet channel 606 of FIG. 6A.

Similarly, microfluidic multiplexer circuit 604 can be in fluid communication with a chip interface device outlet port, such as chip interface outlet port 609 of FIG. 6A. As illustrated in FIG. 6A, chip interface device outlet port 609 can be in fluid communication with any with any of fluidic manifold waste lines 416A-416B through chip interface device outlet channel 608. Fluidic interface block 602 of FIG. 11 can have an outlet port for each of a chip waste microchannel or a bulk waste microchannel channel of multiplexer circuit 604, each of which are in fluid communication with a fluidic interface block outlet line connector, such as fluidic interface block outlet line connector 603 of FIG. 11. Each fluidic interface block outlet line connector can be connected to one of an outlet line, such as one of fluidic manifold waste lines 416A-416B of FIG. 1A. In that regard, each waste line is in fluid communication with a waste container, such as waste container 460 of of waste compartment 450 of FIG. 1A.

Accordingly, fluidic interface 610 of chip interface device 600A provides a macro to micro interface between fluidic system input lines and a microfluidic multiplexer circuit, such as between fluidic manifold input lines 414A-414E of fluidic system 400 of FIG. 1A and microfluidic multiplexer circuit 604 of FIG. 11. Similarly, fluidic interface 610 of chip interface device 600A provides a micro to macro interface from microfluidic between a multiplexer and fluidic system output lines, such as between multiplexer circuit 604 of FIG. 11 to fluidic manifold waste lines 416A-416B of fluidic system 400 of FIG. 1A. Moreover, for various cell analysis systems of the present disclosure, an end user can specify the selection of a reagent or solution, as well as the sequence, the flow rate, flow duration, as well as the measurement protocol during the flow of a selected reagent or solution.

In addition to fluidic interface 610, chip interface device 600A can have chip interface device flange 620 of FIG. 11. As previously described herein in reference to FIG. 2, fluid dam 560 is sealed against sensor chip 520 to prevent leakage at the interface between fluid dam 560 and sensor chip 520 during analysis, thereby preventing the shorting of electrical connections between sensor chip 520 and chip device substrate 510, as well as other operational electrical connections of sensor array device 500 to a pogo pin block and a PC board. Chip interface device flange 620 is configured to sealably and reversibly engage a chip device, such as chip device 500, thereby ensuring the integrity of electrical components of a sensor array device and associated electronic components during use.

Chip interface device flange 620 of chip interface device 600A can include flange rim 622 on flange outer surface 624, upon which flange rim O-ring groove 623 are formed. Once chip interface device 600A is engaged with and reversibly coupled to chip device 500 through chip interface device flange 620, chip device interface bottom surface 626 becomes a top surface of a flow cell, such as flow cell 645 of FIG. 6B, that is formed between chip device interface bottom surface 626 and chip device 500. As previously described herein, a chip interface device inlet port and a chip interface device outlet port, such as chip interface device inlet port 607 and chip interface device outlet port 609 of FIG. 6A, are on chip device interface bottom surface 626. During use, chip interface device inlet port and chip interface device outlet port are in fluid communication with microfluidic multiplexer circuit 604 in a fluidic circuit including the flow cell formed via engagement of chip interface device 600A with chip device 500.

Chip interface device flange 620 can engage with and reversibly couple to fluid dam 560, so that flange rim O-ring 625 is seated below chamfer 562 and against the sealing surface of fluid dam inner wall surface 564 to provide a reversable seal between chip interface device flange 620 of chip interface device 600A and a chip device 500. Once reversibly sealed, chip device interface bottom surface 626 is functionally a flow cell cover that defines a flow cell or analysis chamber, such as flow cell 645 of FIG. 6B, with a defined height of between about 30 $\mu$m to about 770 $\mu$m. The flow cell height can be adjusted using chip interface device spacer 650, with first surface 651, which can be disposed upon chip interface device second surface 614, and with second surface 653, which can be disposed upon fluid dam top or upper surface 566. By using a chip interface device spacer of various thickness, the flow cell height can be adjusted to 770 $\mu$m, providing flow cell volumes of between about 10 $\mu$l to about 310 $\mu$l.

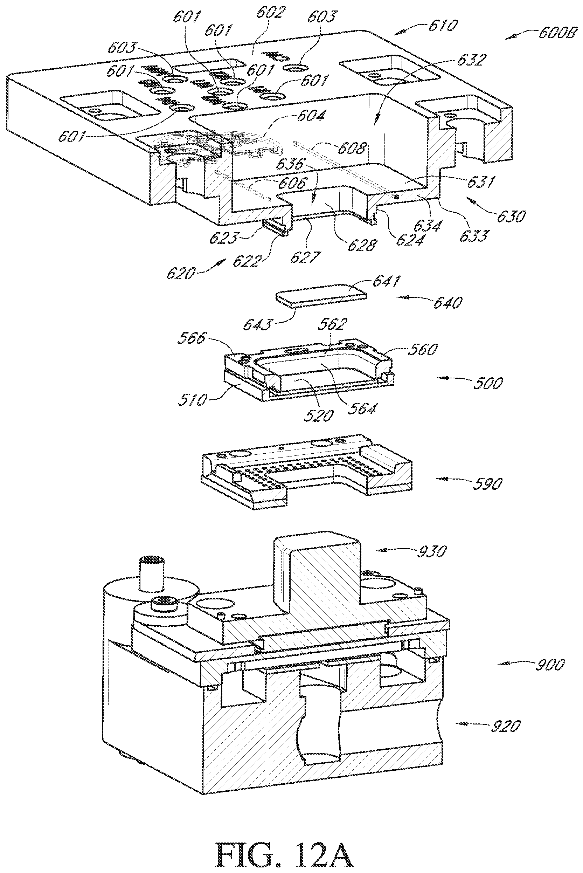

FIG. 12A is an exploded section view that illustrates generally chip interface device 600B of the present disclosure in relationship to chip device 500 as they would be aligned during an analysis. As previously described herein, chip interface device 600B can be integrated with clamping assembly 1000B as depicted in FIG. 8A and FIG. 9A, as well as clamping assembly 1000C as depicted in FIG. 10A through FIG. 10C. Clamping assembly 1000B, as depicted in FIG. 8A and FIG. 9A, as well as clamping assembly 1000C as depicted in FIG. 10A through FIG. 10C, can be integrated with cell analysis system 2000 of FIG. 1A, which can have a fluidic system, such as fluidic system 400 of FIG. 1A and 1B. Chip interface device 600B of FIG. 12A can include fluidic interface 610, chip interface device flange 620 and optical interface 630. In comparison to chip interface device 600A, fluidic interface 610 of chip interface device 600B has been adapted as a fluidic interface to chip device 500 through optical interface 630, so that chip interface device flange 620 is pendent from optical interface 630.

As depicted in FIG. 12A, fluidic interface 610 includes fluidic interface block 602, which can be in fluid communication with microfluidic multiplexer circuit 604. In turn microfluidic multiplexer circuit 604 can be in fluid communication with a chip interface device inlet port and a chip interface device outlet port, such as chip interface device inlet port 607 of FIG. 8A. As shown in FIG. 8A, chip interface device inlet port 607 can be in fluid communication with any of fluidic manifold input lines 414A-414E through chip interface device inlet channel 606. Fluidic interface block 602 of FIG. 12A can have a fluidic interface block inlet port for each reagent or solution contained in a reagent or solution container of a fluidic system, such as solution and reagent containers 420A-420E, 430 and 440 of FIG. 1A. As depicted in FIG. 12A, a plurality of fluidic interface block input line connectors 601 are shown, each of which can be connected to one of an input line, such as any of fluidic manifold input lines 414A-414E shown in FIG. 1A. In that regard, each fluidic system line is in fluid communication with a fluidic interface block inlet port, each of which in turn are in fluid communication with a microfluidic circuit branch of microfluidic multiplexer circuit 604. Each microfluidic circuit branch of microfluidic multiplexer circuit 604 can be selectively placed in fluid communication chip interface device inlet channel 606 of FIG. 12A.

Similarly, microfluidic multiplexer circuit 604 can be in fluid communication with a chip interface device outlet port, such as chip interface outlet port 609 of FIG. 8A. As illustrated in FIG. 8A, chip interface device outlet port 609 can be in fluid communication with any with any of fluidic manifold waste lines 416A-416B through chip interface device outlet channel 608. Fluidic interface block 602 of FIG. 12A can have an outlet port for each of a chip waste microchannel or bulk waste microchannel channel of multiplexer circuit 604, each of which are in fluid communication with a fluidic interface block outlet line connector, such as fluidic interface block outlet line connectors 603 of FIG. 12A. Each fluidic interface block outlet line connector can be connected to one of an outlet line, such as one of fluidic manifold waste lines 416A-416B of FIG. 1A. In that regard, each waste line is in fluid communication with a waste container, such as waste container 460 of of waste compartment 450 of FIG. 1A.

Accordingly, similarly to chip interface device 600A, fluidic interface 610 of chip interface device 600B provides a macro to micro interface between fluidic system input lines and a microfluidic multiplexer circuit, such as between fluidic manifold input lines 414A-414E of fluidic system 400 of FIG. 1A and microfluidic multiplexer circuit 604 of FIG. 12A. Similarly, fluidic interface 610 of chip interface device 600B provides a micro to macro interface between a multiplexer and fluidic system output lines, such as between multiplexer circuit 604 of FIG. 12A to fluidic manifold waste lines 416A-416B of fluidic system 400 of FIG. 1A.

However, unlike chip interface device 600A, chip interface device inlet channel 606 and chip interface device outlet channel 608 of chip interface device 600B are in fluid communication with chip interface device flange 620, which is pendent from optical interface 630. As previously described for chip interface device 600A of FIG. 11, chip interface device flange 620 of FIG. 12A is configured to sealably and reversibly engage a chip device, such as chip device 500, thereby ensuring the integrity of electrical components of a sensor array device and associated electronic components during use. Unlike chip interface device 600A of FIG. 11, chip interface device flange 620 of FIG. 12A is configured to have optically transparent flow cell cover 640 sealably joined to chip interface device flange 620 to further ensure the integrity of electrical components of a sensor array device and associated electronic components.

Chip interface device flange 620 of chip interface device 600B can include flange rim 622 on flange outer surface 624. Flange rim O-ring groove 623 is formed on flange outer surface 624. For chip interface device 600B, chip interface device flange 620 can additionally include flange inner surface 628, which has flange inner surface recess 627. Optically transparent flow cell cover upper surface 641 is glued or otherwise sealably jointed to flange inner surface recess 627. Once chip interface device 600B is engaged with and reversibly coupled to chip device 500 through chip interface device flange 620, optically transparent flow cell cover lower surface 643 becomes a top surface of a flow cell, such as flow cell 645 of FIG. 8B, that is formed between optically transparent flow cell cover 640 and chip device 500.

As previously described herein, a chip interface device inlet port and a chip interface device outlet port, such as chip interface device inlet port 607 and chip interface device outlet port 609 of FIG. 8A, are formed in flange rim 622 of chip interface device flange 620. As depicted in FIG. 12A, chip interface device inlet channel 606 and chip interface device outlet channel 608 of fluidic interface 610 extend through bottom substrate 634 and are in fluid communication with a corresponding microfluidic inlet channel and microfluidic outlet channel formed in chip interface device flange 620 (not shown). The microfluidic inlet channel and microfluidic outlet channel formed in chip interface device flange 620 terminate to form chip interface device inlet port 607 and chip interface device outlet port 609, respectively. During use chip interface device inlet port and chip interface device outlet port are in fluid communication with microfluidic multiplexer circuit 604 of fluidic interface 610 in a fluidic circuit including a flow cell, such as flow cell 645 of FIG. 8B, formed via engagement of chip interface device 600B with chip device 500. As previously described for chip interface device 600A, the flow cell height for chip interface device 600B can be between about 30 $\mu$m to about 770 $\mu$m, and can be adjusted using a spacer, such as spacer 650 of FIG. 11.

Optical interface 630 of chip interface device 600B can include objective recess 632, having an upper surface 631, and bottom surface 633. Optical interface 630 has optical aperture 636 formed through bottom substrate 634. As depicted in FIG. 9B, during use, a microscope objective, such as microscope objective 1110 of FIG. 9B, can be positioned over optically transparent flow cell cover 640.

Additionally depicted in FIG. 12A is chip thermal control assembly 900 with cooling assembly 920. As will be described in more detail herein, chip thermal control assembly 900 can be used for cooling a chip device in any of chip clamp assembly 1000A, chip clamp assembly 1000B, or chip clamp assembly 1000C of the present disclosure.

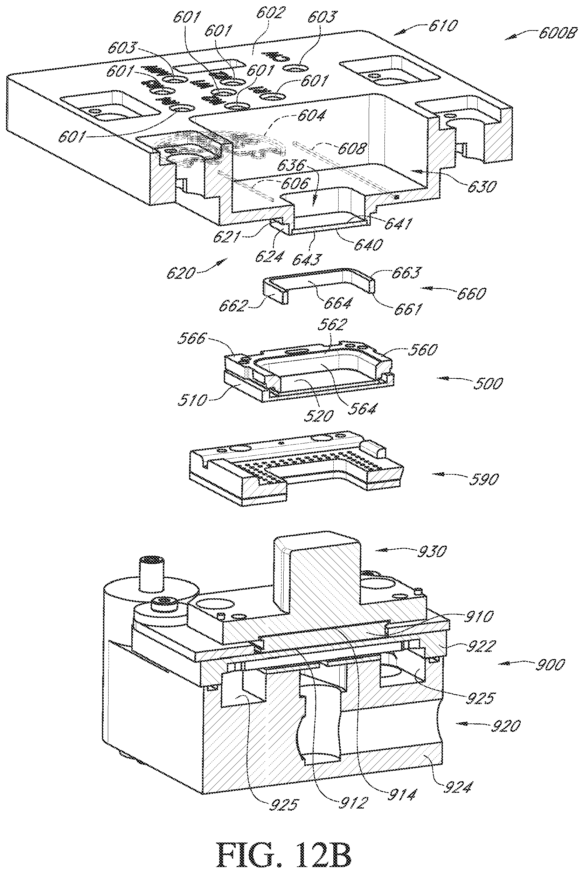

FIG. 12B is an exploded section view that illustrates generally chip interface device 600C of the present disclosure in relationship to chip device 500 as they would be aligned during an analysis. Chip interface device 600C can be integrated with clamping assembly 1000B as depicted in FIG. 8A and FIG. 9A, as well as clamping assembly 1000C as depicted in FIG. 10A through FIG. 10C. Clamping assembly 1000B, as depicted in FIG. 8A and FIG. 9A, as well as clamping assembly 1000C as depicted in FIG. 10A through FIG. 10C, can be integrated with cell analysis system 2000 of FIG. 1A, which can have a fluidic system, such as fluidic system 400 of FIG. 1A and 1B. Chip interface device 600C has many of the same features as previously described herein for chip interface device 600B of FIG. 12A.

For example, fluidic interface 610 of chip interface device 600C includes fluidic interface block 602, which can be in fluid communication with microfluidic multiplexer circuit 604. In turn, microfluidic multiplexer circuit 604 can be in fluid communication with a chip interface device inlet port and a chip interface device outlet port, such as chip interface device inlet port 607 of FIG. 8A. As shown in FIG. 8A, chip interface device inlet port 607 can be in fluid communication with any of fluidic manifold input lines 414A-414E through chip interface device inlet channel 606. Fluidic interface block 602 of FIG. 12A can have a fluidic interface block inlet port for each reagent or solution contained in a reagent or solution container of a fluidic system, such as solution and reagent containers 420A-420E, 430 and 440 of FIG. 1A. As depicted in FIG. 12A and FIG. 12B, a plurality of fluidic interface block input line connectors 601 are shown, each of which can be connected to one of an input line, such as any of fluidic manifold input lines 414A-414E shown in FIG. 1A.

Similarly, on the outlet side, microfluidic multiplexer circuit 604 can be in fluid communication with a chip interface device outlet port, such as chip interface outlet port 609 of FIG. 8A. As illustrated in FIG. 8A, chip interface device outlet port 609 can be in fluid communication with any with any of fluidic manifold waste lines 416A-416B through chip interface device outlet channel 608. Fluidic interface block 602 of FIG. 12A can have an outlet port for each of a chip waste microchannel or bulk waste microchannel channel of multiplexer circuit 604, each of which are in fluid communication with a fluidic interface block outlet line connector, such as fluidic interface block outlet line connectors 603 of FIG. 12A and FIG. 12B.

As depicted in FIG. 12B, optical interface 630 of chip interface device 600C includes optical aperture 636. Additionally, chip interface device 600C includes chip interface device flange 620, which can include flange outer surface 624 and flange sealing edge 621. For chip interface device 600C, chip interface device flange 620 can additionally include flange inner surface 628, onto which flow cell cover upper surface 641 of optically transparent flow cell cover 640 is sealably joined, for example, as described for FIG. 12A. Once chip interface device 600C is engaged with and reversibly coupled to chip device 500 through chip interface device flange 620, flow cell cover lower surface 643 becomes a top surface of a flow cell, such as flow cell 645 of FIG. 8B, that is formed between optically transparent flow cell cover 640 and chip device 500.

With respect to attachment and of sealing chip interface device 600C with chip device 500, as previously described herein, a clamping force is applied to sealably engage a sensor array device with an interface device, as well as engaging a chip device with a pogo pin block and thermal control assembly. For chip interface device 600C, face seal lower surface 661 of face seal 660 can be seated upon sensor chip 520 of chip device 500, with face seal outer surface 662 positioned against the sealing surface of fluid dam inner wall surface 564. When chip interface device 600C is sealably engaged with and reversibly coupled to chip device 500, flange outer surface 624 retains face seal 660 by contact with face seal inner surface 664 , while flange sealing edge 621 applies a vertical compression force to face seal upper surface 663, thereby providing sealing of face seal lower surface 661 against sensor chip 520.

As previously described for chip interface device 600A of FIG. 6A and FIG. 11, and for for chip interface device 600B of FIG. 8A and FIG. 12A, chip interface device 600C is configured to sealably and reversibly engage a chip device, such as chip device 500, thereby ensuring the integrity of electrical components of a sensor array device and associated electronic components during use. In that regard, though chip interface device 600C is described for a chip interface device with optical interface, flange features including flange outer surface 624, and flange sealing edge 621, as well as face seal 660 described for chip interface device 600C can also be used for chip interface device 600A of FIG. 6A and FIG. 11.

Face seal 660 can be formed from materials as described for sample divider 580 of FIG. 4A. As such, face seal 660 can be formed from any variety of natural or synthetic polymeric materials that are chemically inert material and resistant to swelling in aqueous environment, and capable providing a conformal face seal. In that regard, candidate polymer materials can have a durometer rating of 0-60 on the Shore A scale, or 20-90 on the Shore OO scale. Some exemplary materials include, for example, but not limited by, silicone polymers, such as polydimethylsiloxane (PDMS), thermoplastic polyurethanes, thermoplastic Styrene-Butadiene-Styrene (SBS) polymers, such as Sofprene T®, and thermoplastic vlucanizates, such as Santoprene™,

Additionally depicted in FIG. 12B is chip thermal control assembly 900. As previously described herein, chip thermal control assembly 900 can be used for cooling a chip device in any chip clamp assembly 1000A, chip clamp assembly 1000B, or chip clamp assembly 1000C of the present disclosure.

Accordingly, exemplary chip thermal control assembly 900 of FIG. 12B is depicted with thermoelectric cooling (TEC) device 910 mounted on cooling assembly 920, with first TEC surface 912 in contact with TEC cooling assembly 920. Second surface 914 of TEC device 910 is in contact with chip device cooling block 930, which during use of a chip device is in contact with device substrate lower surface 504. Thermal vias (see FIG. 6A and FIG. 8A) in chip device substrate 510 provide effective thermal contact between chip device cooling block 930, chip device substrate 510 and sensor chip 520. As previously described herein, a TEC cooling assembly of the present disclosure can be mounted to the bottom of a chip clamp base, such as chip clamp base 800 with springs 916 of FIG. 6A and FIG.8 to exert sufficient force on chip thermal control assembly 900 to make effective thermal contact with chip device 500.

As such, with the effective thermal contact, chip thermal control assembly 900 can effectively thermally control chip device 500. As depicted in FIG. 12B, cooling assembly 920 can include top plate 922, which is contact with with first TEC surface 912. Thermal control assembly top plate 922 together with thermal control assembly base 924 form thermal control assembly coolant channels 925 for the circulation of coolant through cooling assembly 920. In reference to FIG. 1A, the flow rate of coolant can be controlled by valve board 200, which is controlled by processing electronic assembly 100, where the flow rate of coolant is a factor in dissipating heat from first TEC surface 912, thereby maintaining a temperature of second TEC surface 914 at a defined setpoint. The cooling capacity of chip thermal control assembly 900 is 400W using flow rates of coolant, for example, of 4.5 L/min at 20 °C, provides effective dissipation of heat from TEC 910, which generates 4-5 watts under use.

Regarding the fabricated and assembly of various chip interface devices of the present disclosure, for chip interface device 600A of FIG. 11, chip interface device 600B of FIG. 12A, and chip interface device 600C of FIG. 12B, fluidic interface block input line connectors 601 and fluidic as depicted interface block output line connectors 603 are female connectors capable of accepting any of a standard flangeless male fitting for 1/16" outer diameter (OD) tubing. Tubing used, for example, for fluid lines, such as fluidic manifold input lines 414A-414E and fluidic manifold output lines 416A and 416B of FIG. 1A, can be of any polymeric material that is chemically resistant, stable to pH across a range of 3-9, resistant to swelling in organic solvents, and thermally stable. Exemplary tubing materials include polyether ether ketone (PEEK), as well as various fluorocarbon-based polymeric materials, such as perfluoralkoxy alkane (PFA), fluorinated ethylene propylene (FEP), and ethylene tetrafluoroethylene (ETFE). Tubing can be, for example, 1/16" outer diameter (OD) tubing with an inner diameter (ID) of between 10 mil and 40 mil.

For chip interface device 600A of FIG. 11, chip interface device 600B of FIG. 12A, and chip interface device 600C of FIG. 12B, the devices can be made of a variety of machinable thermoplastic polymers that chemically resistant, stable to pH across a range of 3-9, resistant to swelling in organic solvents, and thermally stable. Exemplary materials for a chip interface device of the present disclosure include polyetherimide (PEI), polyether ether ketone (PEEK), acrylonitrile butadiene styrene (ABS), and polyoxymethylene (POM).

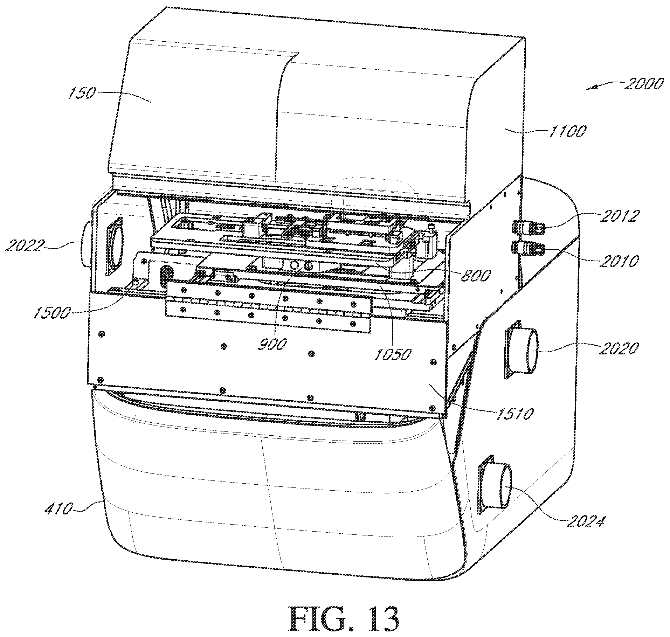

FIG. 13 is a perspective view that illustrates generally cell analysis system 2000 of the present disclosure, which can have analysis compartment door 1510 that can provide ready access to analysis compartment 1500. Cell analysis system 2000 of FIG. 13 can all of the features previously described herein for FIG. 1A and FIG. 1B. By way of non-limiting examples, cell analysis system 2000 of FIG. 13 can have fluidic system (not shown), such as fluidic system 400 of FIG. 1A and FIG. 1B, analysis compartment 1500, which can house chip clamp assembly 1000. As previously described herein, components of chip clamp assembly 1000 as depicted in FIG. 13 include chip interface device 600, chip clamp base 800, chip thermal control assembly 900. As such, chip interface device 600A mounted on chip clamp base 800 of, which can be integrated with clamping assembly 1000A as depicted in FIG. 6A and FIG. 7, can be integrated with cell analysis system 2000 of FIG. 13. Additionally, chip interface device 600B and 600C, which can be integrated with clamping assembly 1000B as depicted in FIG. 8A and FIG. 9A, as well as clamping assembly 1000C as depicted in FIG. 10A through FIG. 10C, can be integrated with cell analysis system 2000 of FIG. 13. A cell analysis system including a microscope assembly can include chip clamp assembly positioning system 1050 for the X,Y positioning of chip interface device 600 relative to a microscope objective (see FIG. 9B), which is a part of microscope assembly 1100 of FIG. 1A.

User display 150 can allow interactive input to cell analysis system 2000 by an end user. Chip thermal control assembly inlet port 2010 is in controllable fluid communication with an inlet port of a chip thermal control assembly. Coolant maintained at a specified temperature can be controllably circulated through thermal control assembly coolant channels, such as thermal control assembly coolant channels 925 of chip thermal control assembly 900 of FIG. 13 and then output to the coolant circulation source through chip thermal control assembly outlet port 2012. The use of a closed thermal control system, such as chip thermal control assembly 900 eliminates use of alternative chip thermal control assemblies that would exhaust heat into the controlled environment of analysis compartment 1500.

Regarding the environmental control of analysis compartment 1500, a thermistor located proximal to chip interface device 600 continuously monitors air flow through analysis compartment 1500, which is data that is fed to a control system for maintaining environment of analysis compartment 1500 at a specified temperature and humidity. Accordingly, air controllably maintained at a specified temperature can be circulated into analysis compartment 1500 through analysis compartment inlet duct 2020 and then output to the air circulation source through analysis compartment outlet duct 2022. Various control systems for maintaining the environment of analysis compartment 1500 at a specified temperature can maintain a temperature over a range of between about 32°C to 60°C within +/- 0.5 °C. Similarly, air flow can be circulated into reagent and solution compartment 410 through reagent and solution compartment inlet duct 2024 and then output to an air circulation source through reagent and solution compartment outlet duct (not shown). Air flow circulated through reagent and solution compartment 410 can be monitored and controlled as described for the environmental control of analysis compartment 1500. Various control systems for maintaining the environment of reagent and solution compartment 410 at a specified temperature can maintain the temperature in a range of between about 10°C to about 50 °C; moreover a selected temperature range of between about 20°C to about 37 °C, which is a range in which many cell-based assays are conducted.

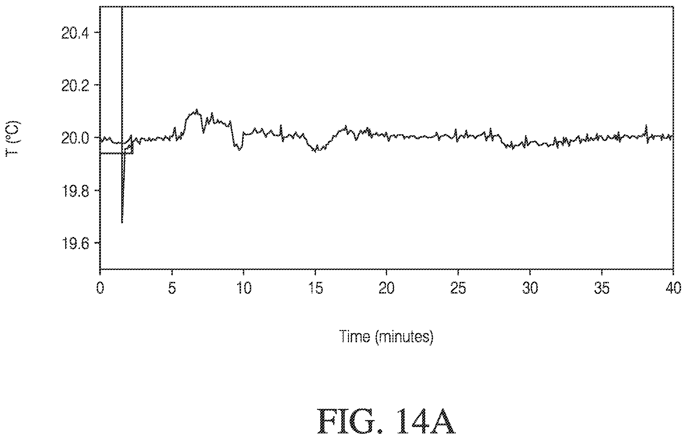

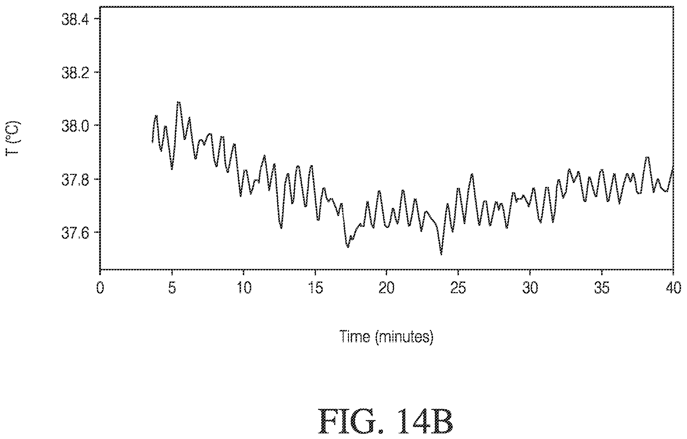

With respect to effectiveness of system thermal control, FIG. 14A is a graph monitoring the recorded temperature of chip device cooling block 930, which is in direct thermal contact with chip device 500, over the course of an experiment using a cell analysis system of the present disclosure. The graph of FIG. 14A demonstrates the ability of chip thermal control assembly 900 to maintain a constant cooling block temperature within +/-0.1 °C. FIG. 14B is a graph taken over the same comparative time course as shown in FIG. 14A, but monitoring effluent from the flow cell at a flow rate of 40-60 μl/minute for an analysis compartment equilibrated to 37 °C. The graph of the thermal profile of the effluent in FIG. 14B demonstrates that the average temperature of a reagent or solution used in a cell analysis system of the present disclosure can be maintained within +/- 0.3 °C

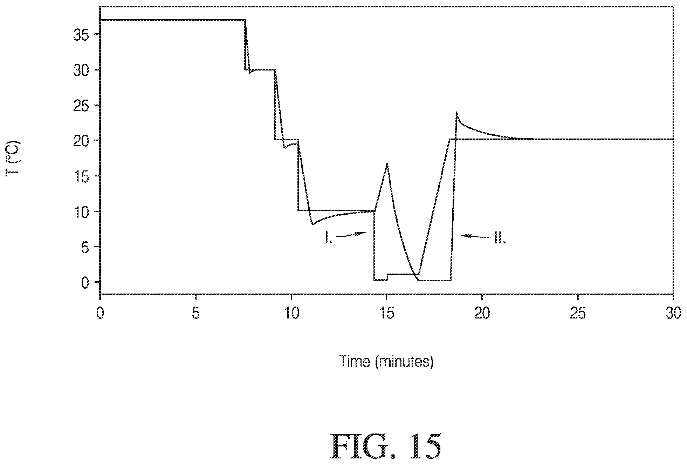

With respect to responsiveness of thermal control of sensor chip 500, FIG. 15 is a set temperature versus time graphs comparing the time course of target setpoint temperatures of a TEC (I.) versus measured temperatures of the TEC (II.). For the measurements taken for the graph of II., data was recorded from a thermistor embedded in a chip device cooling block, such as chip device cooling block 930 of FIG. 12A, and proximal to the cooling surface of the TEC, such as TEC second surface 914 of FIG. 12A. The comparative graphs of FIG. 15 demonstrate that the temperature of a TEC as measured proximal to the cooling surface can reach the desired set point in a response time of 10-150 seconds.

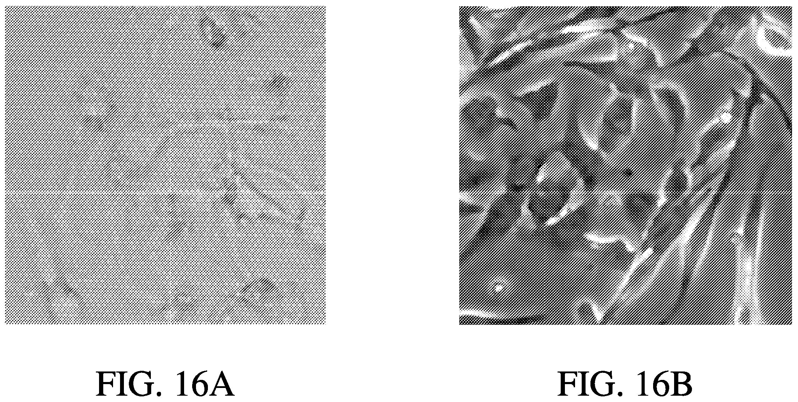

FIG. 16A and FIG. 16B are comparisons of micrographs of cells from an immortalized human hepatic cell line (HEPG2 cells; ATCC catalogue number ATCC-HB-8065). The HEPG2 cell line is used in cell biology as a model for liver hepatocellular carcinoma, and among other applications is also used for studies of cellular metabolism, toxicology, LDL and cholesterol import. In FIG. 16A, the sensor array device on which the HEPG2 cells were plated is an ISFET chip device 1 as shown in Table 1, selective for sensing hydrogen ions, and had no microwells, as depicted for chip device 500A of FIG. 5B. A sensor array device of the present disclosure, such as sensor array device 500 of FIG. 2, has an open structure, thereby allowing the HEPG2 cells to be cultured on the sensor surface in ~1 mL of cell culture media. The device was sterilized with 70% Ethanol in water, rinsed and prepared with 100 μg/mL poly-D-lysine in phosphate buffered saline prior to adding cells to facilitate cell adhesion to a chip device surface as depicted in FIG. 5B.

For plating on the sensor array device, the HEPG2 cells were harvested from culture, resuspended at $2.5 \times 10^5$ cells per mL in complete cell culture medium (composed of Minimum Essential Medium, MEM, Thermo Fisher Scientific catalog number 11095080) plus 10% Fetal Calf Serum (FCS, Thermo Fisher Scientific Catalog number A3160401) and plated onto the sensor array device for overnight incubation in a cell culture incubator under standard conditions at 37 ° C with 95% humidity and 5% carbon dioxide in room air atmosphere. It has been observed that this results in between about 2000 to about 20,000 cells plated on a sensor array surface of a sensor device. Cells were then allowed to adhere to the prepared surface of the sensor array device and grow overnight or longer before microscopic analysis of morphology and qualitative assessment of viability.

As the sensor array device is not optically clear to allow conventional transmission light microscopy, the HEPG2 cells were viewed using a EVOS® M7000 light microscope that was reconfigured as an upright microscope, and facilitated reflected light imagery of cells cultured on the sensor surface. FIG. 16B is a phase contrast image from parallel cultures of HEPG2 cells on a traditional cover glass, also coated with poly-D-lysine and treated identically through the workup and acquisition of the images. Both images were acquired at 10X magnification.

The micrographs of FIG. 16A and FIG. 16B show healthy cultures of HEPG2 cells of normal morphology and viability on both the sensor array device surface (left) and the cover glass (right). Accordingly, cells can be imaged on a sensor array device surface during or after an analysis on a cell analysis system of the present disclosure, such as cell analysis system 2000 of FIG. 1A. Though it is noted that the sensor array device surface scatters the oblique light normally incident from the phase ring illumination, the cells can be clearly observed on top of the sensor array device surface. Also visible in FIG. 16A are the array of hexagonal top metal layers, which are sensor plates with a sensing surface, such as sensor plate 532 of sensor chip 520 of FIG. 5B, which has a sensing surface 526S. Given the known pitch of the pixels, as provided in as shown in Table 1 with reference to FIG. 5B, the pixel grid can be used to estimate the size of cells or cell features. As previously described herein, changes in the surface potential of the sensing surface resulting from cell activity can be used to monitor changes in, for example, cell electrophysiology and metabolism for cells subjected to any of a variety of conditions or stimuli.

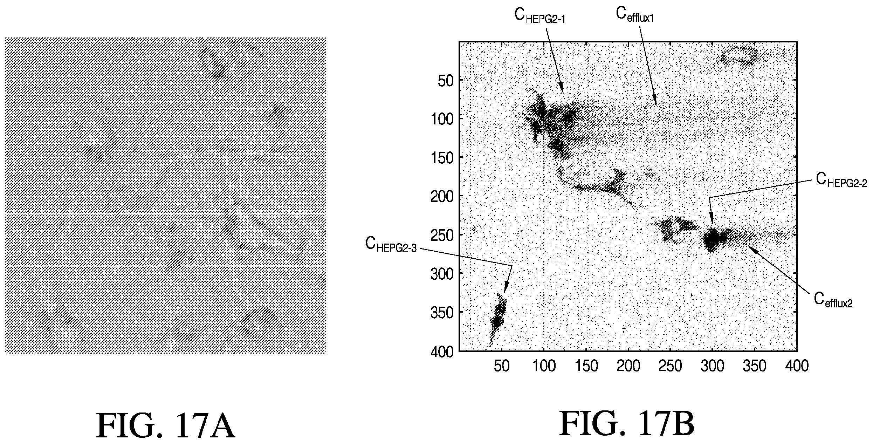

In that regard, FIG. 17A and FIG. 17B are comparisons of the micrograph of HEPG2 cells on an exemplary sensor array device (left) as shown in FIG. 16A in comparison to an electroscopic image of HEPG2 cells on a sensor array device (FIG. 17B). The sensor array device used for the cell analysis from which the electroscopic image on the right was taken was an ISFET chip device 2 of Table 1, selective for sensing hydrogen ions, with microwells, as depicted for chip device 500B of FIG. 5C. The preparation of the cells for plating and the preparation of the device was otherwise as reported for FIG. 17A for the micrograph of the cells on the right.

For the experiment in which the electroscopic image of FIG. 17B was taken, the mitochondrial protonophore FCCP [carbonyl cyanide-4-(trifluoromethoxy) phenylhydrazone] was tested at a concentration of 100 $\mu$M, which is a concentration known to elicit responses leading to cell death. During the application of FCCP, cellular responses were observed demonstrating the ability of cell analysis systems and methods of the present disclosure to visualize the efflux of cellular contents into the laminar flow system. This is depicted for two cells in FIG. 17B, $C_{HEPG2-1}$ and $C_{HEPG2-2}$, for which the the efflux of cellular contents is indicated by $C_{efflux1}$ and $C_{efflux2}$, respectively. Notably, not all cells demonstrated the efflux activity, as depicted in FIG. 11B for $C_{HEPG2-3}$. These results indicate that cell analysis systems and methods of the present disclosure can further discriminate between acute, novel phenotypes exhibited in response to toxin treatment.

As such, the present inventors have recognized that any change in the state of a cell that can cause a change in potential of a sensing surface of a sensor can be monitored by sensors of various sensor array devices of the present disclosure. For example, the present inventors have recognized that a cell is capacitively coupled to the sensing surface of a sensor, so that as the electrical potential across a cell membrane changes in response to a chemical or electrical stimulus, the changing electrical potential across the cell membrane can be detected by sensors of various sensor array devices of the present disclosure. Additionally, any change in cell metabolism or function that can cause a change in potential of the sensing surface of a sensor can be detected by sensors of various sensor array devices of the present disclosure. For example, according to the present disclosure, a sensing surface of a sensor can detect charged species transported across a cell membrane that change the potential of the sensing surface of a sensor. Moreover, it is clear from inspection of the electroscopic image of FIG. 17B, that such changes in cellular function can be locally detected by a change in potential of a sensing surface of sensors either in association with an area of contact or footprint of a cell anchored on a sensor array surface or in areas not associated with a cell footprint, for example, such as for cellular efflux that may stream from a cell in response to a condition or stimulus, such as in response to FCCP.

Though the microscopic images of FIG. 16A/16B and FIG. 17A/17B were either reflected light or phase contrast, fluorescence microscopy is a powerful tool for evaluating various aspects of cell function using any number of probe dyes. Accordingly, different information can be obtained and correlated between data from a light microscopic image of cells on a sensor array device with data from an electroscopic image of the cells on the same sensor array surface. The present inventors have recognized that cell analysis systems of the present disclosure can be used in research or diagnostic testing to interrogate subsets of cells carrying a unique genotype or phenotype among a greater population of untransformed or native cells, or accessory cells from a primary tissue source.

As such, subpopulation studies provided by cell analysis systems of the present disclosure allow the gathering of essential information regarding subpopulations of cells that is unavailable using traditional population level studies of cellular function. Such traditional cell analysis predicated on population level studies are unable, for example, to discriminate the difference between 100 percent of the population responding to a given stimulus with a twofold change in signal, or 50 percent of the population responding with a fourfold change in signal. Further, cell analysis systems of the present disclosure enable the pursuit of analyzing rare cell origins in disease by allowing electroscopically characterized cells to be fixed in place and probed *post hoc* for critical interrogation of mRNA and protein expression profiles, with spatial information kept intact.

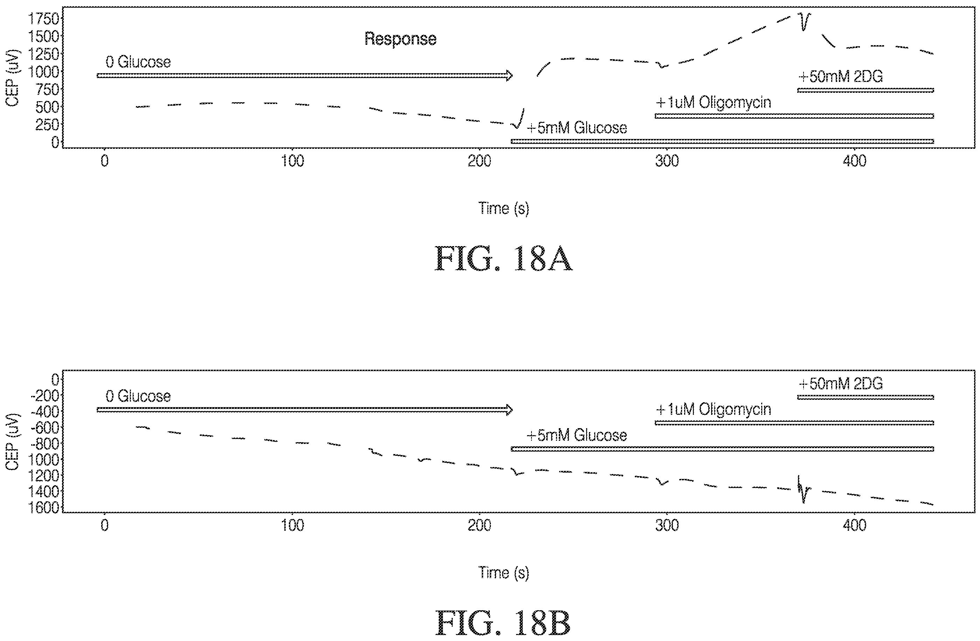

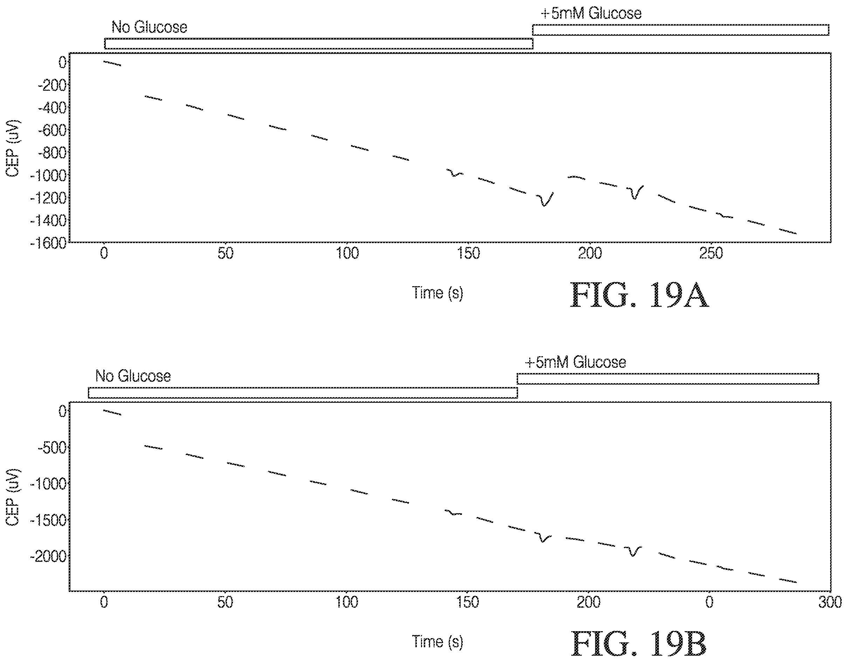

FIG. 18A through FIG. 18B are graphs of data collected during metabolic profiling studies using HEPG2 cells (ATCC-HB-8065), while FIG. 19A and FIG. 19B are graphs of data collected during metabolic profiling studies using A673 cells (ECACC 85111504). FIG. 18A through FIG. 18B are temporal response graphs of counts equivalent to protons (CEP) in microvolts ($\mu$V) versus time. As will be described in more detail herein, the pharmacological dissection of metabolic profiling can be performed to understand the contribution of oxidative metabolism and glycolytic metabolism to metabolic profiling. Accordingly, chemical agents, such as oligomycin, antimycin A, rotenone and metformin can used to understand a contribution of oxidative metabolism to cellular response in a metabolic profiling study, while chemical agents, such as 2-deoxy-D-glucose, koningic acid, fasentin, and AM2394 can used to understand a contribution of glycolytic metabolism to cellular response in a metabolic profiling study.

The chip devices used in the studies were ISFET chip devices selective for sensing hydrogen ions, and were chip devices such as chip device 1 of 1 of Table 1. Given that the analyses performed were for metabolic profiling, chip devices with microwells were selected as depicted for chip device 500B of FIG. 5C. For the studies of FIG. 18A through FIG. 19B, each chip device was prepared and handled in the same fashion from chip device surface coating through incubation and maintenance of the cells in a traditional cell culture incubator.

Prior to seeding the devices with cells, each chip device in open format, such as open format chip device 500 of FIG. 2, and were soaked for 30 minutes with 70% ethanol in water, then rinsed with PBS and then rinsed twice with 1.5mL of a cell culture medium, for example, Dulbecco's Modified Essential Medium (DMEM; Gibco™ catalog # 11995-065), Minimal Essential Medium (MEM; Gibco™ catalog # 11095-080) or McCoy's 5A modified medium (Gibco™ catalog # 12330-031). All serum-based media additionally contain 10% fetal calf serum (Gibco™ catalog # 16000-036). After rinsing with the cell culture medium, the devices were ready for addition to each device of a suspension of cells at a density between 5,000 to 30,000 cells in 1 to 1.5 mL of a cell culture medium, pH 7.4. It has been observed that this results in between about 2000 to about 20,000 cells plated on a sensor array surface of a sensor device. Once seeded, the chip devices were maintained under sterile conditions in a traditional cell culture incubator at 37 °C and 5% $CO_2$ atmosphere. The cells were allowed to grow overnight or longer in these conditions.

Just prior to conducting the studies as presented in FIG. 18A through FIG. 19B, the cells were subjected to a glucose-restriction pretreatment using nutrient assay medium (Agilent catalog # 103575-100; Seahorse XF DMEM Medium, pH 7.4) without glucose. In each chip device prepared with cells for control experiments, the cell culture medium was exchanged with glucose-free nutrient assay medium containing 0.1% ethanol [FIG.18A and FIG. 19A]. In each chip device prepared with cells additionally subjected to koningic acid during pretreatment, the cell culture medium was exchanged with glucose free nutrient with 5 $\mu$M koningic acid (heptelidic acid) that was diluted 1:1000 into nutrient assay medium from a 5mM stock solution in ethanol.[FIG.18B and FIG. 19B]. As will be discussed in more detail herein, koningic acid is a known specific and irreversible inhibitor of the glyceraldehyde phosphate dehydrogenase (GADPH) enzyme, which is an essential enzyme component of the glycolytic pathway. Cultures were left at 37°C in open air atmosphere conditions for two to three hours to starve the cells of glucose prior to placing them in a cell analysis system.

After the glucose restriction pretreatment, a chip device was inserted into a cell analysis system, such as cell analysis system 2000 of FIG. 13, utilizing chip clamp assembly 1000C of FIG. 10A-FIG. 10C. As such, a chip device was connected to a chip interface device, such as chip interface device 600B of FIG. 8A, FIG. 10A-FIG. 10C and FIG. 12A, and chip interface device 600C of FIG. 12B. Once a chip device had been connected to a chip interface device, a flow cell was formed, such as flow cell 645 of FIG. 6B and FIG. 8B. The flow cell formed is in fluid communication with a fluidic system, such as fluidic system 400 of FIG. 1A and FIG. 1B through the fluidic interface of a chip interface device, such as fluid interface 610 of FIG. 11, FIG. 12A and FIG. 12B.

Additionally, the analysis compartment, such as cell analysis compartment 1500 of FIG. 1A and FIG. 13, reagent and solution compartment, such as reagent and solution compartment 410 of FIG. 1A and FIG. 13, as well as the chip device, were maintained at a temperature of between 34°C and 37°C, using an environmental control system as described for FIG. 13, and thermal control assembly 900, such as described for FIG. 6A, FIG. 8A, FIG. 11, FIG. 12A and FIG. 12B.

The study using HEPG2 cells is represented by the graphs of FIG. 18A and FIG. 18B. Regarding the graph of FIG. 18A, after the glucose restriction period, as previously described herein, at the initiation of the study using the first chip device, nutrient assay medium was introduced through the flow cell for an initial equilibration period of a little over 200 seconds. After the initial equilibration, a series of solutions were sequentially introduced. First, HEPG2 cells on the chip device were reintroduced to solution of glucose, followed by introduction of a solution of oligomycin, which is a known inhibitor of ATP synthetase in mitochondria, then by a solution of 2-deoxy-D-glucose, which is known to competitively inhibit glucose metabolism. As indicated in the graph of FIG. 18A, at 220 seconds, a 5mM glucose solution in nutrient assay medium was introduced through the flow cell, then at 295 seconds, a solution of 1 $\mu$M oligomycin in nutrient assay medium was introduced through the flow cell, and then at 371 seconds, a 50 mM 2-deoxy-D-glucose solution in nutrient assay medium was introduced through the flow cell. The graph of FIG. 18A represents an averaged temporal response of counts equivalent to protons (CEP) in microvolts ($\mu$V) versus time for HEPG2 cells from a selected region of interest of a population of cells. For the experiment represent by FIG. 18A, the region of interest contained a population of 33 cells of a total of 7968 cells identified on the sensor array.

The experiment represented by FIG. 18B was performed with a second chip device prepared in the same fashion as previously described. In contrast to the experiment represented by FIG. 18A, just prior to inserting the chip device in the cell analysis system, for the study of FIG. 18B, the cell culture medium was exchanged with glucose free nutrient assay medium containing 5 $\mu$M koningic acid and left at 37°C in open air atmosphere conditions for two to three hours. Once the experiment represented by FIG. 18B was initiated, it was conducted in the same fashion as described for the experiment of FIG. 18A. The selected region of interest The graph of FIG. 18B represents an averaged temporal response (CEP-$\mu$V versus time) for HEPG2 cells from the same selected region of interest as was selected for FIG. 18A. For the experiment represent by FIG. 18B, the region of interest contained a population of 77 cells of a total of 3936 cells identified on the sensor array.

Regarding FIG. 18A, for cells pretreated for the control experiments without koningic acid during glucose restriction, an uptick in signal is observed upon reintroduction of glucose. As can be discerned by inspection of FIG. 18A, the uptick in signal after the reintroduction of glucose was potentiated by introduction of oligomycin, which decouples mitochondrial respiration thereby decoupling oxidative metabolism. The signal potentiated with the introduction of was then attenuated by the sequential introduction of 2-deoxy-D-glucose, which competes for glucose entry and inhibits hexokinase in glycolysis thereby decoupling glycolytic metabolism.

In contrast, as depicted in FIG. 18B, cells on the second chip device that were subjected to 5 $\mu$M koningic acid during the glucose restriction period showed no change in the averaged signal from cells sampled during the course of the study. For the study represented by FIG. 18B, the oligomycin and 2-deoxy-D-glucose effects demonstrated in the control study represented by FIG. 18A were abolished.

The results of these studies indicate that the responses observed in the control study of FIG. 18A were demonstrably glycolytic in origin, and track real time changes within the microdomain of the cell's footprint as glycolytic metabolic activity is recruited into activity upon reintroduction of glucose following a withdrawal period. Moreover, that the response is glycolytic in nature is borne out in the combination of the results of FIG. 18A, showing an potentiation of signal upon introduction of oligomycin, indicating no untoward impact in response by decoupling oxidative metabolism, followed by signal decrease with 2-deoxyglucose, indicating a response by decoupling glycolytic metabolism. That the response is glycolytic in nature is additionally borne out by the results of FIG. 18B, showing the abolition of response to glucose reintroduction with cells pretreated during glucose restriction with koningic acid.

With respect to what is presented in FIG. 18A, various protocols using ChemFET-based cell analysis systems can initiate an experiment with cells that have not previously been subjected glucose restriction, and as such maintained in standard cell nutrient medium and nutrient assay medium, which include 5 mM glucose. Accordingly, cells so prepared can be subjected to pharmacological dissection of metabolic profiling to understand the contribution of oxidative metabolism and glycolytic metabolism to metabolic profiling.

In that regard, chemical agents, such as oligomycin, antimycin A, rotenone and metformin can used to understand a contribution of oxidative metabolism to cellular response in a metabolic profiling study, each having a different mechanism of action on mitochondrial oxidative phosphorylation. For example, as previously described here, oligomycin is a known inhibitor of ATP synthetase. Additionally, antimycin A binds to cytochrome c reductase, thereby impacting the Q-cycle of complex III, rotenone inhibits the NADH-ubiquinone reductase, blocking availability of oxygen for cellular respiration, and metformin inhibits mitochondrial complex I. Further, chemical agents, such as 2-deoxy-D-glucose, koningic acid, fasentin, and AM2394 can used to understand a contribution of glycolytic metabolism to cellular response in a metabolic profiling study. As previously described, 2-deoxy-D-glucose is known to inhibit, for example, hexokinase, while koningic acid is an inhibitor of the GADPH enzyme. Additionally, fasentin inhibits glucose uptake by inhibiting glucose transporter 1 and glucose transporter 4, while AM2394 is a glucose kinase activator, thereby acting to stimulate glycolytic metabolism.

The impact of using agents for metabolic profiling for cells maintained under standard conditions is demonstrated in FIG. 18A. In FIG. 18A, with the addition of glucose, glycolytic metabolic activity is recruited into activity, effectively establishing a new baseline. The sequential introduction of oligomycin as an agent decoupling oxidative metabolism, followed by the introduction of 2-deoxy-D-glucose as an agent decoupling glycolytic metabolism, is exemplary of a label-free metabolic profiling protocol enabling an understanding of the contribution of oxidative metabolism and glycolytic metabolism in cell analysis.

FIG. 19A and FIG. 19B are graphs of data collected during time course studies using A673 cells prepared and inserted into a cell analysis system as previously described for the HEPG2 cell studies of FIG. 18A and FIG. 18B. For the studies using the A673 cells, only with the glucose reintroduction challenge was performed.

In that regard, for the study represented the graph of FIG. 19A, after the glucose restriction period as previously described herein, at the initiation of the study using the first chip device, nutrient assay medium was introduced through the flow cell for an initial equilibration period of about 179 seconds. After the initial equilibration, a 5mM glucose solution in nutrient assay medium was introduced through the flow cell. The graph of FIG. 19A represents an averaged temporal response (CEP-$\mu$V versus time) from a selected region of interest for a population of 158 A673 cells from a total of 7872 cells observed on the sensor array.

The experiment represented by FIG. 19B was performed with a second chip device prepared in the same fashion as previously described. In contrast to the experiment represented by FIG. 19A, just prior to inserting the chip device in the cell analysis system, for the study of FIG. 19B, the cell culture medium was exchanged with glucose free nutrient assay medium containing 5 $\mu$M koningic acid and left at 37°C in open air atmosphere conditions for two to three hours. Once the experiment represented by FIG. 19B was initiated, it was conducted in the same fashion as described for the experiment of FIG. 19A. The graph of FIG. 19B represents an averaged temporal response (CEP-$\mu$V versus time) for A673 cells from the same selected region of interest as was selected for FIG. 19A. For the experiment represented by FIG. 19B, the region of interest contained a population of 155 cells from a total of 6144 cells identified on the sensor array.

As can be discerned through comparison of FIG. 19A and FIG. 19B to FIG. 18A and FIG. 18B, the results of the glucose restriction and glucose reintroduction with and without koningic acid were comparable to the results using HEPG2 cells. Further, the glucose restriction and glucose reintroduction assays of the present disclosure have also been performed on human skeletal myoblast (Thermo Fisher catalog # A12555) with comparable results to those presented for HEPG2 and A673 cells.

After completion of each experiment represented by FIG. 18A and FIG. 18B for the HEPG2 cells, and by FIG. 19A and FIG. 19B for the A673 cells, control microscopy studies using calcein acetoxymethyl (AM) ester, a known fluorescent indicator of live cells, were performed. In each case, the results showed that koningic acid treated cells suffered no acute toxicity from the treatment and had identical calcein staining profiles in comparison to control vehicle treated cultures of FIG. 18A and FIG. 19A. These control microscopy studies confirmed that what was observed using the ChemFET-based cell analysis system to perform the studies represented by FIG. 18A through FIG. 19B for both HEPG2 and A673 cells were observed using live cells.

Therefore, what is presented in FIG. 18A and FIG. 18B for HEPG2 cells, and FIG. 19A and FIG. 19B for A673 cells represents label-free metabolic profiling using ChemFET devices involving responses from living cells. It should be understood that while the results of the studies presented are demonstrably glycolytic in origin, label-free metabolic profiling studies using the protocol for glucose restriction, and glucose reintroduction with and without koningic acid as previously described herein are protocols that can be performed more generally for evaluation of the contribution of oxidative metabolism and glycolytic metabolism to metabolic profiling.

Additionally, the pharmacological dissection of metabolic profiling can be performed in protocols with and without a glucose restriction pretreatment to understand the contribution of oxidative metabolism and glycolytic metabolism to metabolic profiling. Chemical agents, such as oligomycin, antimycin A, rotenone, and metformin can used to understand a contribution of oxidative metabolism to cellular response in a metabolic profiling study, while chemical agents, such as 2-deoxy-D-glcose, koningic acid, fasentin, and AM2394 can used to understand a contribution of glycolytic metabolism to cellular response in a metabolic profiling study. Such measurements have the potential to lend powerful mechanistic insights to the field of bioenergetics, where metabolic profiling of cells is performed in standard testing of cell health, cancer phenotype and mitochondrial respiratory chain activity.

Accordingly, what is depicted in the graphs of FIG. 18A through FIG. 19B demonstrate the sensitivity of label-free measurement for cells in populations, for example, of dozens to hundreds of cells. In that regard, these results suggest that cell numbers needed to characterize a drug, gene, or nutrient panel on cellular bioenergetics could potentially be miniaturized from tens of thousands per sample to possibly, for example, dozens of cells, hundreds of cells, or possibly thousands of cells per sample. The impact of such miniaturization can potentially support larger scale studies of metabolic profiling in research and pharmaceutical discovery cell biology.

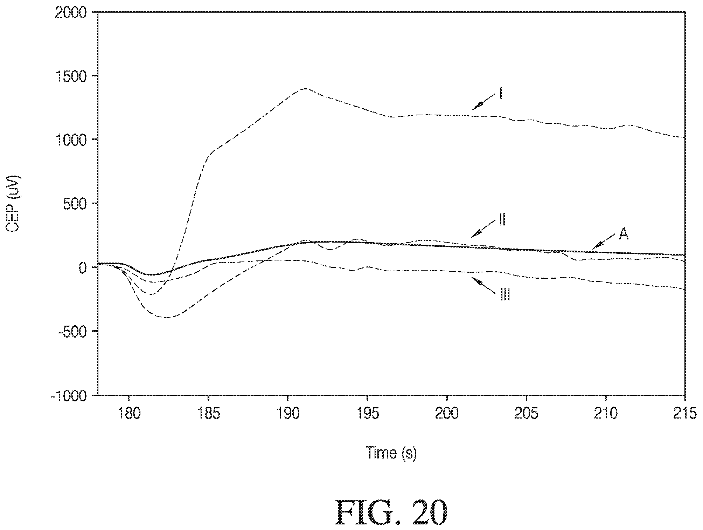

FIG. 20 is a temporal response graph (CEP-$\mu$V versus time) from a selected time interval of the study presented in FIG. 19A, which displays responses of 3 individual cells (Roman numerals I, II, and III) versus the average population response (A).

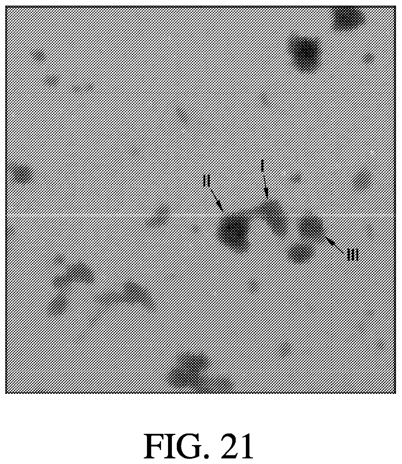

FIG. 21 is an electroscopic image displaying the location of the cells on the chip device. The electroscopic image of FIG. 21 was obtained automatically at the completion of each the experiment by flowing a solution having a pH step of 0.1 pH unit lower pH than the pH of the nutrient assay medium through the flow cell. For example, for the experiments represented by FIG. 18A-19B, a solution the nutrient assay medium at pH 7.3 was used. As previously described herein, changes in the surface potential of the sensing surface of a ChemFET sensor result in an output signal from the sensor. In this example, where the sensor is an ISFET sensor selective for sensing hydrogen ions, each sensor is effectively a pH sensor. When the solution of lower pH is passed over the surface, the sensors covered by the footprint of a cell and not in direct contact with the solution therefore have a different response profile than sensors in direct contact with the solution of lower pH. The intensity of the response, displayed in gray scale in the electroscopic image of FIG. 21, provides a clear image of the footprint of each cell. Location of the cells on a chip can be determined using electroscopic image segmentation for segmenting the electroscopic image data into one or more cell regions corresponding to locations of the cells on the sensor array surface and one or more background regions corresponding to corresponding to areas on the sensor array having no cells based on characteristics of the signal samples generated in response to the step change in pH.

Recalling, FIG. 19A provides the graph of the average response of 158 cells. Additionally, as presented in the graph of FIG. 20, ChemFET-based cell analysis systems of the present disclosure can discriminate and track the responses of individual cells. Accordingly, ChemFET-based cell analysis systems of the present disclosure can discriminate a range of individual cell responses in a population of cells on a chip device. For example, in FIG. 20, the graph displays a response that is distinctively large versus the population average (A), such as the response rendered by trace of the response of cell I of FIG. 20, a response that reflects the population average, such as the response rendered by trace of the response of cell II of FIG. 20, as well as a response that is lower than the population average, such as the response rendered by trace of the response of cell III of FIG. 20.

As such, ChemFET-based cell analysis systems of the present disclosure can allow an end user to see whether or not the responses of various subpopulations of cells are lost in the population average response, or to see how a subpopulation of cells may carry the response window in a measurement if the response of the subpopulation is sufficiently large.

Moreover, as provided in the electroscopic image of FIG. 21, the individual cells associated with each response can be located on a chip device. Accordingly, cell analysis systems of the present disclosure provide the temporal recording of individual cell responses, as well as the electroscopic image and location of a cell on a chip device for any time over the course of an experiment. In that regard, Chem-FET based cell analysis systems can allow an end user to pursue analysis of cells having a range of response profiles by allowing electroscopically characterized cells to be fixed in place and probed *post hoc,* for example, for mRNA and protein expression profiles.

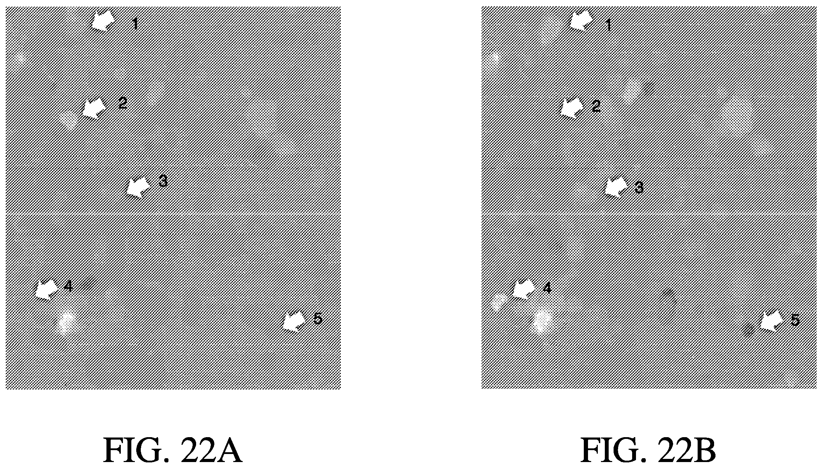

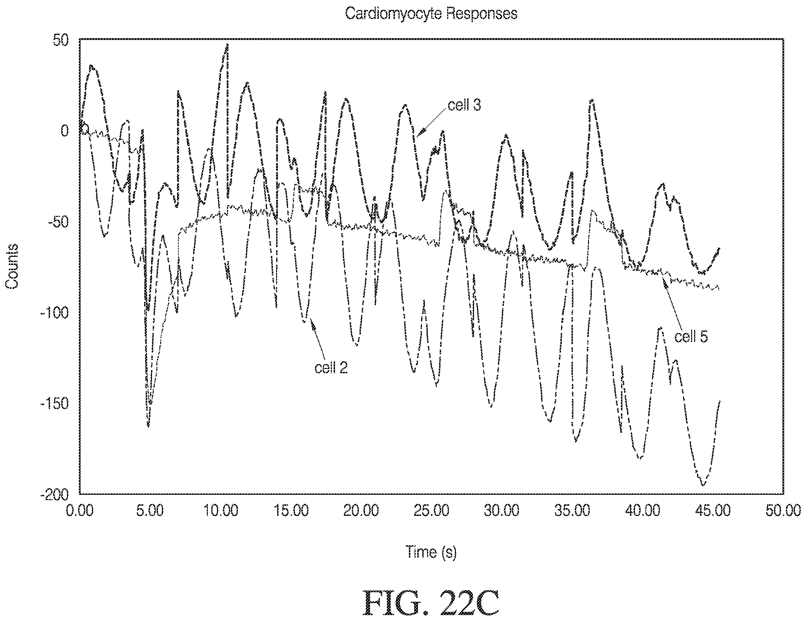

FIG. 22A and FIG.22B are electroscopic images of cardiomyocytes at two sequential time points, while FIG. 22C is the graph of responses for 3 cells selected from the electroscopic images of FIG. 22A and FIG. 22B. The cells of the studies represented by FIG. 22A through FIG. 22C are induced pluripotent stem cell (iPS) derived human cardiomyocytes Fujifilm Cellular Dynamics catalog # R1218). The iPS cardiomyocytes were grown on chip devices for extended time periods one to four weeks. As will be described in more detail herein, the plated iPS cardiomyocytes remained healthy and viable, and displayed spontaneous activity, which was monitored electroscopically for activity.

**The** iPS cardiomyocytes were processed according to manufacturer's instructions and plated onto chip devices at a density of 25,000 viable cells per mL in manufacturer's plating media. It has been observed that this results in between about 2000 to about 20,000 cells plated on a sensor array surface of a sensor device. The chip devices were ISFET chip devices selective for sensing hydrogen ions, and were chip devices such as chip device 1 of 1 of Table 1. Given that the study was electrophysiological observation of beating cardiomyocytes, chip devices with no microwells as depicted for chip device 500A of FIG. 5B were used. For the studies of iPS cardiomyocytes presented in FIG. 22A through FIG. 22C, each chip device was coated with fibronectin. Prior to seeding the devices with cells, each chip device in open format, such as open format chip device 500 of FIG. 2, was prepared with a fibronectin surface coating. To prepare chip devices with a fibronectin coating, the sensor array device surface was soaked for 30 minutes with 70% ethanol in water, then rinsed with PBS before adding a solution of 5μg/mL fibronectin, which was allowed to sit for two hours. The devices were then rinsed twice with 1.5 mL of a cell culture medium, for example, iPS cardiomyocyte maintenance medium (Gibco™ catalog #A2920801).

The chip devices seeded with iPS cardiomyocytes cells were placed in a 5% CO2 incubator at 37 °C and allowed to settle overnight before a complete media change. Following this, manufacturer's maintenance medium was exchanged at 50% vol/vol every 48 hours for three weeks before recording activity from the cells on a ChemFET-cell analysis system and components as previously described herein for the studies represented by FIG. 18A through FIG. 22. Once inserted into a cell analysis system, the plated iPS cardiomyocytes cells were perfused through the flow cell with a 5 mM glucose solution in nutrient assay medium, as previously described herein for the studies represented by FIG. 18A through FIG. 22.

Spontaneous activity could be observed from mature iPS human cardiomyocytes during viewing of electroscopic video recordings. For the purpose of patent illustration, FIG. 22A and FIG. 22B are two frames from an electroscopic video recording from time points separated by 2 seconds that illustrate the spontaneous activity of 5 selected cells. For example, signal intensity for cell #1, cell # 3, cell # 4 and cell #5 is greater at the later time point of FIG. 22B than signal intensity for the cells as shown in FIG. 22B. Additionally, signal intensity for cell #2 is diminished at the later time point of FIG. 22B than signal intensity for the cell as shown in FIG. 22B. The spontaneous excursions from basal electroscopic signal observed among the differentiated iPS cardiomyocyte cells may be electrogenic or metabolic in origin, but are consistent with parallel observations of rhythmic contraction and calcium ion fluxes that can be readily observed with traditional white light and fluorescence microscopy with standard calcium indicator dyes.

Figure 22C is a graphic display of oscillatory bioelectric activity for three cells of FIG. 22A and FIG. 22B. The individual traces represent an averaged signal coming from ISFET sensors underneath the footprints of cells 2, 3 and 5 of FIG. 2A and Figure 22B. These traces capture graphically the oscillatory bioelectric activity that the electroscopic video made visually apparent. In FIG. 22C, spontaneous bioelectric activity can be seen as oscillations in the electroscopic signal intensity from a subpopulation of the cells in the preparation, indicating successful differentiation.

Measuring the spontaneous and triggered beating responses from differentiated cardiomyocytes in culture remains a standard in preclinical drug safety testing, profiling, and discovery. In some examples, sufficient cell numbers have been grown to support large scale compound library screening campaigns, usually carried out in population measurements of fluorescence output from fluorogenic voltage or calcium ion sensing dye systems in High Throughput Screening (HTS) modalities. With the ability to noninvasively monitor cardiac activity from single cells or cells in a population, electroscopic imaging allows scientists to passively monitor healthy, diseased or drug-altered activity from vastly smaller numbers of cells than traditional cardiac safety profile testing.

Further, there is mechanistic insight within the signals obtained during the noninvasive, cell level recordings of activity that are possible with electroscopic imaging. While a traditional calcium ion indicator dye can faithfully report target engagement proximal to the flux, release, binding or exchange of calcium in cells, the holistic electrical and metabolic output of healthy, beating cardiomyocytes recorded in electroscopic imaging will afford insight to a far greater range of potential drug and gene interactions than those limited to the single output of calcium. As a tool used in the phenotyping of cells, one of the greatest advantages to electroscopic imaging is the rich diversity of membrane and bioenergetic protein targets and interactions underlying the recorded signal. The high signal output of single cells means that many fewer cells are needed per test in order to generate meaningful population data out of many single cell responses.

While various systems, devices and methods for cell analysis of the present disclosure have been shown and described herein, it will be obvious to those skilled in the art that such systems, devices and methods are provided by way of example only. It is intended that the following claims define the scope of the present invention and that systems, devices and methods within the scope of these claims be covered thereby.

## Claims

1. A sensor device (500) comprising:

   an array of ChemFET sensors (520) mounted on a substrate, wherein the ChemFET sensors have a pitch of between 850 nm to 3.3 $\mu$m and an input capacitance of between 1 fF to 10 fF; and
   a frame (560) mounted on the substrate; the frame having a chamfer (562) and an inner wall surface (564) that is a sealing surface enabling reversible formation of a flow cell.

2. The sensor device of claim 1, wherein the sensor device has a frame rate of between 15 fps to about 120 fps.

3. The sensor device of claim 1, wherein the array of ChemFET sensors is an array of ISFET sensors.

4. The sensor device of claim 3, wherein the ISFET sensors are selective for hydrogen ion.

5. The sensor device of claim 1, wherein the sensor device further comprises an array of microwells capacitively coupled to the sensor array.

6. The sensor array of claim 1, further comprising a removable cover fitting over the frame.

7. The sensor device of claim 1, further including a sample divider for providing isolated areas of the sensor array for plating each of a spatially divided sample of cells.

8. The sensor device of claim 7, wherein the sensor array further comprises a biocompatible surface coating.

9. The sensor device of claim 8, wherein the biocompatible coating is selected from fibronectin, poly-D-lysine, laminin, collagen, extracellular matrix and combinations thereof.

10. The sensor device of any preceding claim, wherein the frame (560) is a fluid dam.

11. The sensor device of any preceding claim, wherein the frame (560) is chemically inert.

12. A flow cell device, comprising:

    a sensor device (500) according to any preceding claim; and
    an interface device (600A, 600B) comprising a fluidic interface (610) and a chip interface device flange (620) for reversibly coupling with the frame (560) and having a bottom surface (626) providing a flow cell cover for defining a flow cell.

13. The flow cell device of claim 12, wherein the chip interface device flange (620) comprises a flange O-ring 625 for seating against the inner wall surface (564).

14. The flow cell device of claim 12 or 13, wherein the fluidic interface comprises a chip interface device inlet channel (606) which terminates as a chip interface device inlet port (607) on the bottom surface (626), and a chip outlet channel (608) which terminates as a chip interface device outlet port (609) on the bottom surface (626).

15. The flow cell device of claim 14, wherein the chip interface device inlet port (607) and the chip interface device outlet port (609) are located diagonally so that fluid flows into one corner and out of an opposite diagonal corner.

16. The flow cell device of claim 14, wherein the chip interface device inlet channel (606) is in fluid communication with an input line (414) and the chip interface device outlet channel (608) is in fluid communication with an output line (416).

**Patentansprüche**

1.  Sensorvorrichtung (500), umfassend:

    eine Anordnung von ChemFET-Sensoren (520), die auf einem Substrat montiert sind, wobei die ChemFET-Sensoren einen Abstand zwischen 850 nm und 3,3 μm und eine Eingangskapazität zwischen 1 fF und 10 fF aufweisen; und
    einen Rahmen (560), der auf dem Substrat montiert ist; wobei der Rahmen eine Fase (562) und eine Innenwandfläche (564) aufweist, die eine Dichtfläche ist, die eine reversible Bildung einer Durchflusszelle ermöglicht.

2.  Sensorvorrichtung nach Anspruch 1, wobei die Sensorvorrichtung eine Bildrate von zwischen 15 fps bis etwa 120 fps aufweist.

3.  Sensorvorrichtung nach Anspruch 1, wobei die Anordnung von ChemFET-Sensoren eine Anordnung von ISFET-Sensoren ist.

4.  Sensorvorrichtung nach Anspruch 3, wobei die ISFET-Sensoren für Wasserstoffionen selektiv sind.

5.  Sensorvorrichtung nach Anspruch 1, wobei die Sensorvorrichtung ferner eine Anordnung von Mikrovertiefungen umfasst, die kapazitiv an die Sensoranordnung gekoppelt sind.

6.  Sensoranordnung nach Anspruch 1, ferner umfassend eine entfernbare Abdeckung, die über den Rahmen passt.

7.  Sensorvorrichtung nach Anspruch 1, ferner beinhaltend einen Probenteiler zum Bereitstellen isolierter Bereiche der Sensoranordnung zum Plattieren jeder von einer räumlich geteilten Probe von Zellen.

8.  Sensorvorrichtung nach Anspruch 7, wobei die Sensoranordnung ferner eine biokompatible Oberflächenbeschichtung umfasst.

9.  Sensorvorrichtung nach Anspruch 8, wobei die biokompatible Beschichtung aus Fibronektin, Poly-D-Lysin, Laminin, Collagen, extrazellulärer Matrix und Kombinationen davon ausgewählt ist.

10. Sensorvorrichtung nach einem vorhergehenden Anspruch, wobei der Rahmen (560) ein Fluiddamm ist.

11. Sensorvorrichtung nach einem vorhergehenden Anspruch, wobei der Rahmen (560) chemisch inert ist.

12. Durchflusszellenvorrichtung, umfassend:

    eine Sensorvorrichtung (500) nach einem vorhergehenden Anspruch; und
    eine Schnittstellenvorrichtung (600A, 600B), die eine fluidische Schnittstelle (610) und einen Chipschnittstellenvorrichtungsflansch (620) zum reversiblen Koppeln mit dem Rahmen (560) umfasst und eine Bodenfläche (626) aufweist, die eine Durchflusszellenabdeckung zum Definieren einer Durchflusszelle bereitstellt.

13. Durchflusszellenvorrichtung nach Anspruch 12, wobei der Chipschnittstellenvorrichtungsflansch (620) einen Flansch-O-Ring 625 zum Sitzen gegen die Innenwandfläche (564) umfasst.

14. Durchflusszellenvorrichtung nach Anspruch 12 oder 13, wobei die fluidische Schnittstelle einen Chipschnittstellenvorrichtungseinlasskanal (606), der als eine Chipschnittstellenvorrichtungseinlassöffnung (607) an der Bodenfläche (626) endet, und einen Chipauslasskanal (608), der als eine Chipschnittstellenvorrichtungsauslassöffnung (609) an der Bodenfläche (626) endet, umfasst.

15. Durchflusszellenvorrichtung nach Anspruch 14, wobei die Chipschnittstellenvorrichtungseinlassöffnung (607) und die Chipschnittstellenvorrichtungsauslassöffnung (609) diagonal angeordnet sind, sodass Fluid in eine Ecke und aus einer gegenüberliegenden diagonalen Ecke strömt.

16. Durchflusszellenvorrichtung nach Anspruch 14, wobei der Chipschnittstellenvorrichtungseinlasskanal (606) in Fluidkommunikation mit einer Eingangsleitung (414) steht und der Chipschnittstellenvorrichtungsauslasskanal (608) in Fluidkommunikation mit einer Ausgangsleitung (416) steht.

**Revendications**

1. Dispositif de capteur (500) comprenant :

   un réseau de capteurs ChemFET (520) montés sur un substrat, lesdits capteurs ChemFET comportant un pas compris entre 850 nm et 3,3 $\mu$m et une capacité d'entrée comprise entre 1 fF et 10 fF ; et
   un cadre (560) monté sur le substrat ; le cadre comportant un chanfrein (562) et une surface de paroi interne (564) qui est une surface d'étanchéité permettant la formation réversible d'une cuve à flux continu.

2. Dispositif de capteur de la revendication 1, ledit dispositif capteur comportant une fréquence de trames comprise entre 15 tps et environ 120 tps.

3. Dispositif de capteur de la revendication 1, ledit réseau de capteurs ChemFET étant un réseau de capteurs ISFET.

4. Dispositif de capteur de la revendication 3, lesdits capteurs ISFET étant sélectifs pour les ions hydrogène.

5. Dispositif de capteur de la revendication 1, ledit dispositif de capteur comprenant en outre un réseau de micropuits couplés de manière capacitive audit réseau de capteurs.

6. Réseau de capteurs de la revendication 1, comprenant en outre un couvercle amovible s'adaptant sur le cadre.

7. Dispositif de capteur de la revendication 1, comprenant en outre un diviseur d'échantillon destiné à fournir des zones isolées du réseau de capteurs pour le placage de chacun d'un échantillon spatialement divisé de cellules.

8. Dispositif de capteur de la revendication 7, ledit réseau de capteurs comprenant en outre un revêtement de surface biocompatible.

9. Dispositif de capteur de la revendication 8, ledit revêtement biocompatible étant choisi parmi la fibronectine, la poly-D-lysine, la laminine, le collagène, la matrice extracellulaire et des combinaisons de ceux-ci.

10. Dispositif de capteur d'une quelconque revendication précédente, ledit cadre (560) étant un barrage de fluide.

11. Dispositif de capteur d'une quelconque revendication précédente, ledit cadre (560) étant chimiquement inerte.

12. Dispositif de cuve à flux continu, comprenant :

    un dispositif de capteur (500) selon une quelconque revendication précédente ; et
    un dispositif d'interface (600A, 600B) comprenant une interface fluidique (610) et une bride (620) de dispositif d'interface de puce pour un couplage réversible avec le cadre (560) et comportant une surface inférieure (626) fournissant un couvercle de cuve à flux continu pour définir une cuve à flux continu.

13. Dispositif de cuve à flux continu de la revendication 12, ladite bride (620) de dispositif d'interface de puce comprenant un joint torique 625 de bride destiné à s'appuyer contre la surface de paroi interne (564).

14. Dispositif de cuve à flux continu de la revendication 12 ou 13, ladite interface fluidique comprenant un canal d'entrée (606) de dispositif d'interface de puce qui se termine sous la forme d'un orifice d'entrée (607) de dispositif d'interface de puce sur la surface inférieure (626), et un canal de sortie (608) de puce qui se termine sous la forme d'un orifice de sortie (609) de dispositif d'interface de puce sur la surface inférieure (626).

15. Dispositif de cuve à flux continu de la revendication 14, ledit orifice d'entrée (607) de dispositif d'interface de puce et ledit orifice de sortie (609) de dispositif d'interface de puce étant situés en diagonale afin que le fluide s'écoule dans un coin et sorte du coin diagonal opposé.

16. Dispositif de cuve à flux continu de la revendication 14, ledit canal d'entrée (606) de dispositif d'interface de puce étant en communication fluidique avec une conduite d'entrée (414) et ledit canal de sortie (608) de dispositif d'interface de puce étant en communication fluidique avec une conduite de sortie (416).

FIG. 1A

FIG. 1B

FIG. 2

**FIG. 3**

FIG. 4A

EP 4 264 248 B1

FIG. 4B

EP 4 264 248 B1

FIG. 5A

EP 4 264 248 B1

FIG. 5B

EP 4 264 248 B1

FIG. 5C

EP 4 264 248 B1

FIG. 6A

EP 4 264 248 B1

FIG. 6B

FIG. 7

FIG. 8A

EP 4 264 248 B1

FIG. 8B

FIG. 9A

FIG. 9B

FIG. 10A

FIG. 10B

FIG. 10C

FIG. 11

FIG. 12A

FIG. 12B

FIG. 13

FIG. 14A

FIG. 14B

FIG. 15

FIG. 16B

FIG. 16A

FIG. 17B

FIG. 17A

FIG. 18A

FIG. 18B

EP 4 264 248 B1

No Glucose

+5mM Glucose

FIG. 19A

Time (s)

No Glucose

+5mM Glucose

FIG. 19B

Time (s)

FIG. 20

EP 4 264 248 B1

FIG. 21

FIG. 22B

FIG. 22A

Cardiomyocyte Responses

FIG. 22C

EP 4 264 248 B1

**EP 4 264 248 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63128751
- US 20200088676 A1